# EUROPEAN PATENT APPLICATION

(11) **EP 4 810 085 A1**
(43) Date of publication of application: **23.09.2026**
(21) Application number: 24923668.8
(22) Date of filing: 25.09.2024
(51) Int. Cl.: A61M 5/32

(54) **MEDICINAL LIQUID ADMINISTRATION DEVICE**

(30) Priority: 07.02.2024 JP 2024017191
(71) Applicant: Terumo Kabushiki Kaisha, Tokyo 151-0072 (JP)
(72) Inventor: OKIHARA, Hitoshi, Fujinomiya-shi, Shizuoka 418-0004 (JP)
(74) Representative: Prüfer & Partner mbB Patentanwälte · Rechtsanwälte
(86) International application number: PCT/JP2024/034181
(87) International publication number: WO 2025/169534

(57) **Abstract**

A medicinal liquid administration device (10A) includes a lock portion (18) that couples a cap (16) provided at a distal end of a housing (12) and the housing (12). The lock portion (18) has a coupling member (42), a first coupling structure (44) that couples the housing (12) and the coupling member (42), and a second coupling structure (46) that couples the cap (16) and the coupling member (42). When a coupling state of at least one of a first coupling structure (44) and a second coupling structure (46) is released, an unlocked state is reached in which the cap (16) can be detached from the housing (12).

## Description

### Technical Field

The present invention relates to a medicinal liquid administration device.

### Background Art

JP 6154061 B1 discloses that a cylindrical casing, a syringe accommodated in the casing and filled with a medicinal liquid, and a cap detachably provided at a distal portion of the casing are provided. When a user uses the device, a puncture needle at a distal end of the syringe protrudes from a distal end of the casing after detachment of the cap from the distal end of the casing, and the puncture needle punctures the skin to subcutaneously administer a medicinal liquid.

### Citation List

### Patent Literature

Patent Literature 1: JP 6154061 B1

### Summary of Invention

### Technical Problem

The above-described medicinal liquid administration device has a configuration in which the puncture needle protrudes from the distal end of the housing and is exposed to the outside after detachment of the cap. Accordingly, it is desired to reduce erroneous puncture in a case where the cap is erroneously removed.

### Solution to Problem

An object of the present invention is to solve the above-described problem.
(1) An aspect of the present invention provides a medicinal liquid administration device for administering a medicinal liquid into a living body, including a housing that is formed in a hollow cylindrical shape, a syringe having a cylindrical body that is accommodated in the housing and filled with the medicinal liquid, and a puncture needle that communicates with the cylindrical body and administers the medicinal liquid into a living body, and a cap that is provided at a distal end of the housing and detached when the puncture needle punctures a puncture target, in which the medicinal liquid administration device includes a lock portion coupling the cap and the housing, the lock portion includes a coupling member, a first coupling structure coupling the housing and the coupling member, and a second coupling structure coupling the cap and the coupling member, and by releasing a coupling state of at least one of the first coupling structure and the second coupling structure, an unlocked state is reached in which the cap can be detached from the housing.
   According to the medicinal liquid administration device, due to the lock portion provided to couple the housing and the cap, the cap is prevented from being detached from the distal end of the housing in a state in which the first coupling structure or the second coupling structure is not released. Accordingly, it is possible to reduce the occurrence of erroneous puncture due to erroneous detachment of the cap from the distal end of the housing.
(2) In the medicinal liquid administration device according to (1), at least a part of the coupling member may be operated in a direction different from an axial direction of the housing to bring the lock portion into the unlocked state.
   Due to this configuration, since the operation direction of the coupling member is different from the axial direction of the housing, the coupling member is less likely to be erroneously operated by a user.
(3) In the medicinal liquid administration device according to (1) or (2), the unlocked state may be reached by releasing a coupling state between the housing and the coupling member by the first coupling structure, and when the cap is detached from the housing, the coupling member may be detached from the housing together with the cap in a state in which the cap and the coupling member are coupled by the second coupling structure.
   Due to this configuration, when the coupling state by the first coupling structure is released to enter the unlocked state, the coupling member is detached together with the cap. Therefore, the second coupling structure does not remain on the housing, and the coupling member can be discarded together with the cap.
(4) In the medicinal liquid administration device according to (1), the coupling member may be a rotary member formed in a cylindrical shape and provided rotatably about an axis of the housing, and the unlocked state may be reached by rotating the rotary member.
   Due to this configuration, the coupling member can be constituted with a simple configuration, and thus the cost can be reduced.
(5) In the medicinal liquid administration device according to (2), the coupling member may be formed in a hollow cylindrical shape having a distal end opening and a proximal end opening, the cap being inserted into the distal end opening and the housing being inserted into the proximal end opening, and may include an internal space in which the cap and the housing are accommodated, the coupling member may have a first rib protruding from an inner wall of a distal portion having the distal end opening, and an engagement groove provided on an inner wall of a proximal portion having the proximal end opening and extending in a circumferential direction, the cap may have a second rib provided at a proximal portion and protruding from a cap outer wall, a distal portion of the housing may have an engagement protrusion protruding from a housing outer wall, the first coupling structure may be an engagement structure in which the engagement protrusion and the engagement groove engage with each other, the second coupling structure may be an abutment structure in which a proximal portion of the first rib and a distal portion of the second rib face each other in the axial direction of the housing, and the proximal portion and the distal portion can abut on each other, and by rotating the coupling member relative to the housing about an axis of the housing, the engagement protrusion of the housing may disengage from the engagement groove of the coupling member, and a first coupling released state may be reached in which a coupling state of the first coupling structure is released.
   Due to this configuration, the first coupling structure can effectively couple the housing and the coupling member by engaging the engagement protrusion with the engagement groove. The coupling by the first coupling structure can be easily released by rotating the coupling member relative to the housing. By causing the first rib and the second rib to abut on each other in the axial direction when the cap is detached from the housing, the cap is prevented from being moved in the distal-end direction relative to the coupling member, and the coupling member can be detached together with the cap.
(6) In the medicinal liquid administration device according to (5), the engagement groove of the coupling member may have a spiral shape centered on an axis of the coupling member, when the coupling state of the first coupling structure is released, the engagement protrusion may move along the engagement groove, so that the coupling member moves relative to the housing in the distal-end direction, and the proximal portion of the first rib and the distal portion of the second rib in the second coupling structure are separated from each other, and when the cap is detached from the housing in the distal-end direction, the proximal portion of the first rib and the distal portion of the second rib may abut on each other.
   Due to this configuration, the coupling state of the first coupling structure can be easily released by rotating the coupling member relative to the housing. When the cap is detached from the housing, the coupling member can be easily detached together with the cap by the second coupling structure.
(7) In the medicinal liquid administration device according to (5), the housing may have a first indicator, the coupling member may have a second indicator, and by rotating the coupling member relative to the housing about the axis of the housing, the first indicator and the second indicator may be linearly disposed in the axial direction of the housing and the coupling member, the engagement protrusion of the housing may disengage from the engagement groove of the coupling member, and the first coupling released state may be reached in which the coupling by the first coupling structure is released.
   Due to this configuration, when the coupling member is rotated relative to the housing, the user can easily visually confirm that the rotation of the coupling member has been completed and the coupling of the first coupling structure has been released through the first and second indicators.
(8) In the medicinal liquid administration device according to (7), the first indicator may be a window portion through which the syringe can be visually confirmed from outside of the housing, and the second indicator may be a hole portion penetrating an outer wall of the coupling member.
   Due to this configuration, by using the window portion provided for confirming the syringe, the manufacturing cost of the medicinal liquid administration device can be reduced compared to a structure in which a new first indicator is provided. By providing the second indicator as the hole portion, the second indicator can be easily provided in the coupling member.
(9) In the medicinal liquid administration device according to (5), the coupling member may have a groove forming portion including the engagement groove, at least the groove forming portion may be transparent, and the engagement groove may be visually confirmed from outside of the coupling member through the groove forming portion.
   Due to this configuration, the user can effectively confirm that the rotation of the coupling member has been completed by confirming the engagement state between the engagement protrusion and the engagement groove from the outside of the coupling member by the transparent groove forming portion.
(10) In the medicinal liquid administration device according to (6), in the first coupling released state in which the engagement protrusion of the housing disengages from the engagement groove of the coupling member and the first coupling structure is released, the cap may have an abutment portion that abuts on the distal portion of the coupling member.
   Due to this configuration, when the coupling by the first coupling structure is released, the user can effectively confirm that the rotation of the coupling member has been completed and the first coupling released state has been reached in which the coupling by the first coupling structure is released, by recognizing that the coupling member has moved in the distal-end direction and its distal portion has abutted on the abutment portion.
(11) In the medicinal liquid administration device according to (1), the coupling member may be a rotary member that is provided across a proximal portion of the cap and a distal portion of the housing and is rotatable about a vertical axis perpendicular to an axial direction of the housing, and the unlocked state may be reached by rotating the rotary member.
   Due to this configuration, the coupling member can be constituted with a simple configuration, and thus the cost can be reduced.
(12) In the medicinal liquid administration device according to (2), in a state in which the housing and the cap are coupled by the first coupling structure and the second coupling structure, the coupling member may be provided across a proximal portion of the cap and a distal portion of the housing, the coupling member may be provided on a cap outer wall to be rotatable about a vertical axis perpendicular to the axial direction of the housing, the distal portion of the housing may have a first engagement protrusion protruding from a housing outer wall, the coupling member may have a first engagement groove with which the first engagement protrusion engages, and the first engagement groove may extend along a rotation direction about the vertical axis, the first coupling structure may be an engagement structure between the first engagement protrusion and the first engagement groove, the second coupling structure may be a fixing structure between the coupling member and the cap, and by rotating the coupling member about the vertical axis, the first engagement protrusion may disengage from the first engagement groove, and a first coupling released state may be reached in which the first coupling structure is released.
   Due to this configuration, when the coupling state by the first coupling structure is released and the cap is detached, the coupling member coupled by the second coupling structure can be detached together with the cap. Therefore, the coupling member can be discarded together with the cap without remaining on the housing. The coupling between the housing and the coupling member by the first coupling structure can be easily released by rotating the coupling member about the vertical axis.
(13) In the medicinal liquid administration device according to (12), the cap may have a second engagement protrusion protruding from the cap outer wall, the coupling member may have a second engagement groove with which the second engagement protrusion engages, and the second engagement groove may extend along the rotation direction about the vertical axis, and by rotating the coupling member about the vertical axis, the second engagement protrusion may move along the second engagement groove.
   Due to this configuration, when the coupling member is rotated about the vertical axis, the rotation of the coupling member is effectively stabilized due to the movement of the second engagement protrusion along the second engagement groove.
(14) In the medicinal liquid administration device according to (13), by rotating the coupling member about the vertical axis, the second engagement protrusion may disengage from the second engagement groove, and a second coupling released state may be reached in which the coupling between the coupling member and the cap by the second coupling structure is released.
   Due to this configuration, when the cap is detached from the housing, the coupling member can be detached from both the housing and the cap.
(15) In the medicinal liquid administration device according to (1), the coupling member may be a slide member that is provided across a proximal portion of the cap and a distal portion of the housing and is slidable in an intersecting direction intersecting an axial direction of the housing, and the unlocked state may be reached by moving the slide member in the intersecting direction.
   Due to this configuration, the coupling member can be constituted with a simple configuration, and thus the cost can be reduced.
(16) In the medicinal liquid administration device according to (2), in a state in which the housing and the cap are coupled by the first coupling structure and the second coupling structure, the coupling member may be provided across a proximal portion of the cap and a distal portion of the housing, the coupling member may be provided on a cap outer wall to be slidable in an intersecting direction that intersects the axial direction of the housing and is along a housing outer wall, the distal portion of the housing may have a first engagement protrusion protruding from the housing outer wall, the coupling member may have a first engagement groove with which the first engagement protrusion engages, and the first engagement groove may extend along the intersecting direction, the first coupling structure may be an engagement structure between the first engagement protrusion and the first engagement groove, the second coupling structure may be a fixing structure between the coupling member and the cap, and by moving the coupling member in the intersecting direction, the first engagement protrusion may disengage from the first engagement groove, and a first coupling released state may be reached in which the first coupling structure is released.
   Due to this configuration, when the coupling state by the first coupling structure is released and the cap is detached, the coupling member coupled to the cap by the second coupling structure can be detached together with the cap. Therefore, the coupling member can be discarded together with the cap without remaining on the housing. The coupling between the housing and the coupling member by the first coupling structure can be easily released by sliding the coupling member in the intersecting direction.
(17) In the medicinal liquid administration device according to (16), the cap may have a second engagement protrusion protruding from the cap outer wall, the coupling member may have a second engagement groove with which the second engagement protrusion engages, and the second engagement groove may extend along the intersecting direction, and by moving the coupling member in the intersecting direction, the second engagement protrusion may move along the second engagement groove.
   Due to this configuration, when the coupling member is slid in the intersecting direction, the movement of the coupling member is effectively stabilized due to the movement of the second engagement protrusion along the second engagement groove.
(18) In the medicinal liquid administration device according to (17), by moving the coupling member in the intersecting direction, the second engagement protrusion may disengage from the second engagement groove, and a second coupling released state may be reached in which the coupling between the coupling member and the cap by the second coupling structure is released.
   Due to this configuration, when the cap is detached from the housing, the coupling member can be detached from both the housing and the cap.
(19) In the medicinal liquid administration device according to (1), the coupling member may be a plate-like member that is provided across a proximal portion of the cap and a distal portion of the housing and is detachable from at least one of the cap and the housing, and the unlocked state may be reached by detaching the plate-like member from one of the cap and the housing.
   Due to this configuration, the coupling member can be constituted with a simple configuration, and thus the cost can be reduced.
(20) In the medicinal liquid administration device according to (2), a distal portion of the housing may have a first engagement protrusion protruding from a housing outer wall, a proximal portion of the cap may have a second engagement protrusion protruding from a cap outer wall, in a state in which the housing and the cap are coupled by the first coupling structure and the second coupling structure, the coupling member may be provided across the proximal portion of the cap and the distal portion of the housing, the coupling member may be a plate-like member having a first engagement hole with which the first engagement protrusion engages and a second engagement hole with which the second engagement protrusion engages, the first coupling structure may be an engagement structure in which the first engagement protrusion and the first engagement hole engage with each other, the second coupling structure may be an engagement structure in which the second engagement protrusion and the second engagement hole engage with each other, and by disengaging the coupling member from the housing, the first engagement protrusion may disengage from the first engagement hole, and a first coupling released state may be reached in which the coupling state by the first coupling structure is released, or by disengaging the coupling member from the cap, the second engagement protrusion may disengage from the second engagement hole, and a second coupling released state may be reached in which the coupling state by the second coupling structure is released.
   Due to this configuration, since the coupling between the coupling member and the housing by the first coupling structure can be released by detaching the coupling member from the housing, and the coupling between the coupling member and the cap by the second coupling structure can be released by detaching the coupling member from the cap, the user can select the handling of the coupling member after the coupling state by the lock portion is released.
(21) In the medicinal liquid administration device according to (20), in the unlocked state, the coupling state of one of the first coupling structure and the second coupling structure may be released, and the coupling state of the other of the first coupling structure and the second coupling structure may be maintained.
   Due to this configuration, by maintaining the coupling state of the other of the first and second coupling structures, it is possible to select whether to detach and discard the coupling member together with the cap or to leave the coupling member attached to the housing.
(22) In the medicinal liquid administration device according to (20), in the unlocked state, both the coupling states by the first coupling structure and the second coupling structure may be released, and the first coupling released state and the second coupling released state may be reached.
   Due to this configuration, when the cap is detached from the housing, the coupling member can be prevented from remaining on either the cap or the housing.
(23) In the medicinal liquid administration device according to (20), the coupling member may have a first slit extending between the first engagement hole and the second engagement hole, and the first slit may connect the first engagement hole and the second engagement hole.
   Due to this configuration, when the first engagement protrusion is disengaged from the first engagement hole, the first slit facilitates the disengagement of the first engagement protrusion, making it easier to detach the coupling member. When the second engagement protrusion is disengaged from the second engagement hole, the first slit facilitates the disengagement of the second engagement protrusion, making it easier to detach the coupling member.
(24) In the medicinal liquid administration device according to (23), the coupling member may include a second slit provided in an opposite direction to the first slit with respect to the first engagement hole, and a third slit provided in an opposite direction to the first slit with respect to the second engagement hole, and the second slit may be formed to be connected to the first engagement hole, and the third slit may be formed to be connected to the second engagement hole.

Due to this configuration, by providing the second and third slits in addition to the first slit, it is possible to more effectively disengage the first and second engagement protrusions from the first and second engagement holes. Accordingly, the coupling member is more easily detached.

### Advantageous Effects of Invention

According to the present invention, the lock portion coupling the cap and the housing is provided, and the lock portion includes the first coupling structure coupling the housing and the coupling member and the second coupling structure coupling the cap and the coupling member. Therefore, by releasing a coupling state of at least one of the first coupling structure and the second coupling structure, an unlocked state is reached in which the cap can be detached from the housing. Accordingly, the cap is prevented from being detached from the distal end of the housing in a state in which the first coupling structure or the second coupling structure is not released. Accordingly, it is possible to reduce the occurrence of erroneous puncture due to erroneous detachment of the cap from the distal end of the housing.

### Brief Description of Drawings

[Fig. 1] Fig. 1 is an external perspective view of a medicinal liquid administration device according to a first embodiment of the present invention.
[Fig. 2] Fig. 2 is a partially exploded perspective view of the medicinal liquid administration device of Fig. 1.
[Fig. 3] Fig. 3 is a cross-sectional view of the medicinal liquid administration device of Fig. 1.
[Fig. 4] Fig. 4 is a first explanatory diagram showing a distal portion of the medicinal liquid administration device.
[Fig. 5] Fig. 5 is a second explanatory diagram showing the distal portion of the medicinal liquid administration device.
[Fig. 6] Fig. 6A is an explanatory diagram showing a locked state of a lock portion. Fig. 6B is an explanatory diagram showing an unlocked state of the lock portion. Fig. 6C is a plan view of the unlocked state shown in Fig. 6B.
[Fig. 7] Fig. 7 is an explanatory diagram showing a state in which a cap is detached from a housing in the unlocked state of the lock portion.
[Fig. 8] Fig. 8 is a cross-sectional view showing a state in which the cap is detached from the medicinal liquid administration device of Fig. 3.
[Fig. 9] Fig. 9 is a cross-sectional view showing a puncturing state of the medicinal liquid administration device of Fig. 8.
[Fig. 10] Fig. 10A is an explanatory diagram showing a locked state of a lock portion according to a modification of the first embodiment. Fig. 10B is an explanatory diagram showing an unlocked state of the lock portion.
[Fig. 11] Fig. 11A is a perspective view showing a distal portion of a medicinal liquid administration device according to a second embodiment of the present invention. Fig. 11B is an exploded perspective view of a lock portion in the medicinal liquid administration device of Fig. 11A.
[Fig. 12] Fig. 12 is a plan view showing the distal portion of the medicinal liquid administration device of Fig. 11A.
[Fig. 13] Fig. 13A is an explanatory diagram showing an unlocked state of the lock portion of the medicinal liquid administration device shown in Fig. 12. Fig. 13B is an explanatory diagram showing a state in which a cap is detached from a distal portion of a housing.
[Fig. 14] Fig. 14A is a perspective view showing a distal portion of a medicinal liquid administration device according to a third embodiment of the present invention. Fig. 14B is an exploded perspective view of a lock portion in the medicinal liquid administration device of Fig. 14A.
[Fig. 15] Fig. 15 is a plan view showing the distal portion of the medicinal liquid administration device of Fig. 14A.
[Fig. 16] Fig. 16A is an explanatory diagram showing an unlocked state of the lock portion of the medicinal liquid administration device shown in Fig. 15. Fig. 16B is an explanatory diagram showing a state in which a cap is detached from a distal portion of a housing.
[Fig. 17] Fig. 17 is a plan view showing a distal portion of a medicinal liquid administration device according to a modification of the third embodiment.
[Fig. 18] Fig. 18A is an explanatory diagram showing an unlocked state of a lock portion of the medicinal liquid administration device shown in Fig. 17. Fig. 18B is an explanatory diagram showing a state in which a cap is detached from a distal portion of a housing.
[Fig. 19] Fig. 19 is a perspective view showing a distal portion of a medicinal liquid administration device according to a fourth embodiment of the present invention.
[Fig. 20] Fig. 20 is a plan view showing the distal portion of the medicinal liquid administration device of Fig. 19.
[Fig. 21] Fig. 21 is an explanatory diagram showing an unlocked state of a lock portion of the medicinal liquid administration device shown in Fig. 20.
[Fig. 22] Fig. 22A is an explanatory diagram showing a state in which a cap is detached from a distal portion of a housing. Fig. 22B is an explanatory diagram showing an unlocked state in which a coupling member is detached from the cap and the housing.

### Description of Embodiments

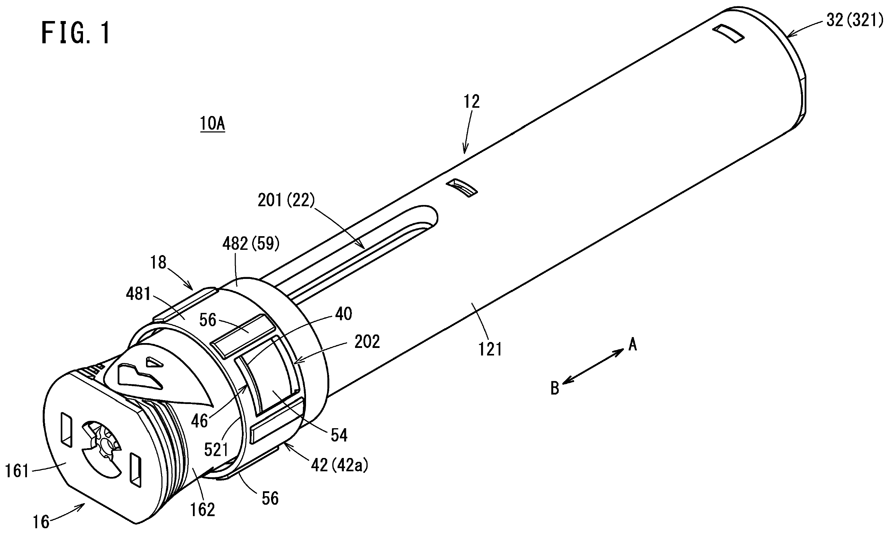

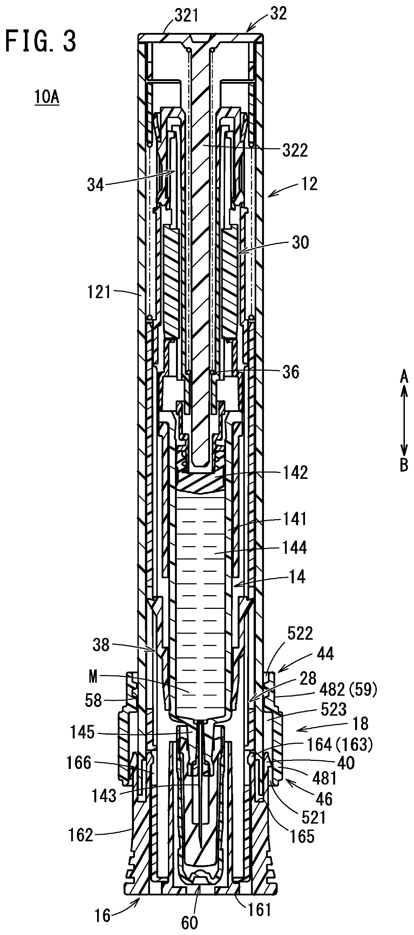

As shown in Fig. 1, a medicinal liquid administration device 10A according to a first embodiment is used to, for example, subcutaneously administer a medicinal liquid M (see Fig. 3) to a patient who is a user. The medicinal liquid administration device 10A includes a housing 12 formed in a hollow cylindrical shape, a syringe 14 (see Fig. 3) accommodated in the housing 12, a cap 16 provided at a distal end of the housing 12, and a lock portion 18 that couples the cap 16 and the housing 12.

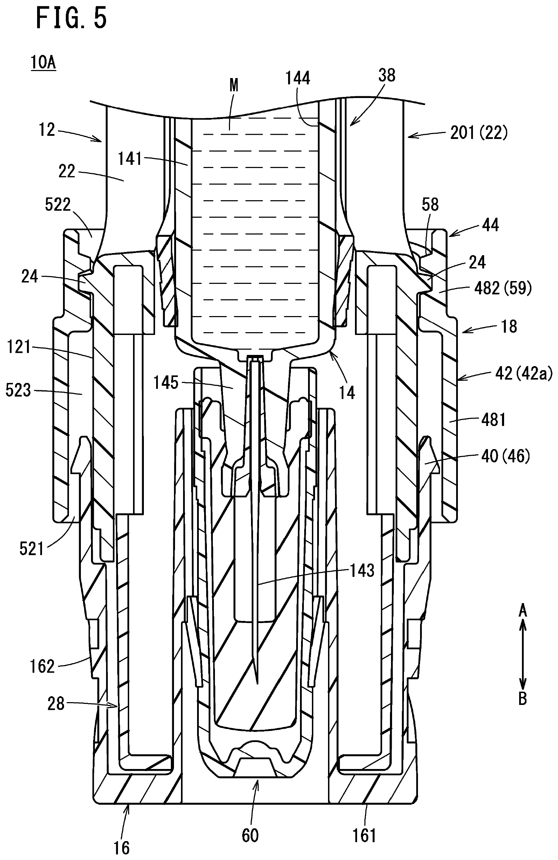

The housing 12 has a predetermined length in the axial direction (direction of the arrows A and B). The housing 12 has an open distal end and an open proximal end (see Fig. 3). A peripheral wall of the housing 12 has a housing outer wall 121. The housing outer wall 121 is provided with a first indicator 201. The first indicator 201 has a plurality of window portions 22 through which the syringe 14 can be visually confirmed from the outside of the housing 12. The plurality of window portions 22 enables the syringe 14 to be visually confirmed from the outside of the housing 12. In the present embodiment, a case where a pair of the window portions 22 is provided will be described. The window portions 22 are not limited to being provided as a pair. One window portion 22 may be provided, or three or more window portions 22 may be provided. The window portion 22 is formed to penetrate the housing outer wall 121 of the housing 12 (see Fig. 5). The medicinal liquid M in the syringe 14 can be confirmed through the window portion 22. The window portion 22 is formed to be long and linear along the axial direction of the housing 12.

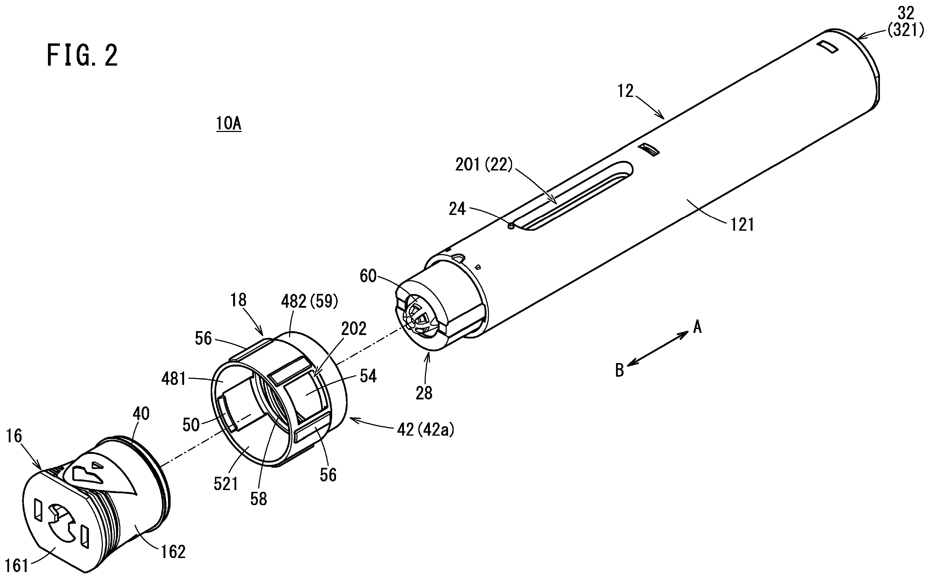

As shown in Fig. 2, the distal portion of the housing 12 includes an engagement protrusion 24 protruding outward from the housing outer wall 121. A pair of the engagement protrusions 24 is provided (see Fig. 5), and each engagement protrusion 24 is disposed between the distal end of each window portion 22 and the distal end of the housing 12. Each engagement protrusion 24 is disposed close to the distal end of the window portion 22 between the distal end of each window portion 22 and the distal end of the housing 12. The engagement protrusions 24 are not limited to being provided as a pair. A configuration may be adopted in which the housing 12 includes a single engagement protrusion 24.

As shown in Fig. 3, a pair of recess portions 166 is formed inside the distal end of the housing 12. The recess portion 166 is recessed radially with respect to an inner circumferential surface of the housing 12. The pair of recess portions 166 is disposed at positions symmetrical with respect to the axis on the inner circumferential surface of the housing 12. When the cap 16 is attached to the distal end of the housing 12, the recess portion 166 is disposed at a position facing a holding arm 163 of the cap 16.

A needle cover 28 and a sleeve body 30 movably accommodated in the housing 12 are accommodated in the housing 12, and the proximal end of the housing 12 is closed by an end cap 32. The needle cover 28 is provided so as to be able to protrude from the distal portion of the housing 12 in the distal-end direction (direction of the arrow B). The sleeve body 30 is formed in a hollow shape and accommodated on the proximal end side of the housing 12. The end cap 32 has a lid portion 321 that blocks the proximal end of the housing 12, and a rod portion 322 extending from the center of the lid portion 321 toward the distal end side (direction of the arrow B). A plunger 34 is provided at the outer circumference of the rod portion 322, and an injection spring 36 is provided between the plunger 34 and the lid portion 321. Due to the resilient force of the injection spring 36, the plunger 34 is biased in the distal-end direction.

The syringe 14 includes a hollow cylindrical body 141, a stopper 142 that is slidably inserted into the cylindrical body 141, and a puncture needle 143 that is provided at a distal end of the cylindrical body 141 and protrudes in the distal-end direction (direction of the arrow B). A cylindrical syringe holder 38 is disposed at the outer circumference of the cylindrical body 141. The cylindrical body 141 is held inside the housing 12 by the syringe holder 38. Examples of the medicinal liquid M to be used include those used for subcutaneous injection to the user (living body).

The cylindrical body 141 is a hollow body and includes a medicine chamber 144 that is filled with the medicinal liquid M. A needle holding portion 145 that holds the puncture needle 143 is provided at a distal end of the cylindrical body 141. The cylindrical body 141 is made of a transparent resin material. The stopper 142 is made of an elastic material such as rubber. The stopper 142 is attached to a distal end of the plunger 34 through a top member (not shown). The stopper 142 is accommodated in the medicine chamber 144 of the cylindrical body 141. The medicinal liquid M in the medicine chamber 144 is sealed in the cylindrical body 141 by the stopper 142.

The puncture needle 143 is a hollow body having a channel through which the medicinal liquid M flows. The puncture needle 143 protrudes from the needle holding portion 145 in the distal-end direction (direction of the arrow B). The channel of the puncture needle 143 communicates with the medicine chamber 144 of the cylindrical body 141 filled with the medicinal liquid M. The medicinal liquid M filling the medicine chamber 144 of the cylindrical body 141 is discharged from a distal end of the puncture needle 143 and administered to the user.

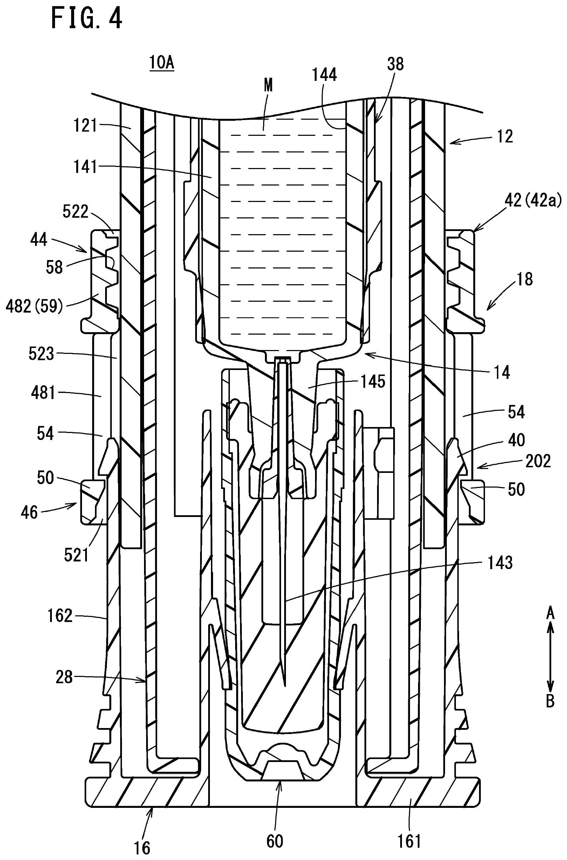

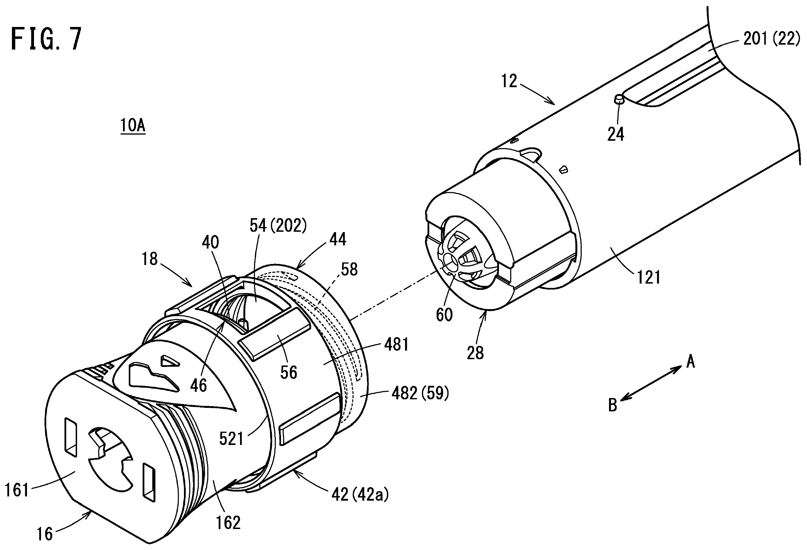

The cap 16 is detached from the distal portion of the housing 12 when the puncture needle 143 punctures a living body (see Fig. 7). As shown in Fig. 4, the cap 16 has a bottomed cylindrical shape, having a bottom wall 161 formed at a distal end and a cap outer wall 162 extending from the bottom wall 161 to the proximal end side (direction of the arrow A). The cap outer wall 162 has an annular shape. A proximal end of the cap 16 is open (see Fig. 3). The cap 16 includes a pair of the holding arms 163 (see Fig. 3) and a cap-side rib 40 (hereinafter, referred to as a second rib 40).

As shown in Fig. 3, the holding arm 163 protrudes from a proximal end of the cap outer wall 162 in the axial direction (direction of the arrow A). The holding arm 163 is disposed radially inward relative to the cap outer wall 162, and is tiltable radially with a coupling part with the cap outer wall 162 as a fulcrum. A hook 164 protruding radially inward is provided at a proximal end of the holding arm 163. The holding arms 163 are disposed as a pair at positions symmetrical to each other across a central axis of the cap 16. The proximal end of the cap 16 has an end insertion groove 165 into which the distal end of the housing 12 can be inserted.

When the cap 16 is attached to the distal end of the housing 12, a proximal end of the cap outer wall 162 abuts on the distal end of the housing 12, and the hooks 164 of the pair of holding arms 163 are engaged with the recess portions 166. The distal end of the housing 12 is inserted into the end insertion groove 165 of the cap 16.

As shown in Fig. 4, the second rib 40 is provided at the proximal portion of the cap 16 and protrudes radially outward from the cap outer wall 162. The second rib 40 has an annular shape along the circumferential direction of the cap outer wall 162 (see Fig. 2). The second rib 40 is provided radially outward from the holding arm 163. An outer surface of the second rib 40 is tapered in the proximal-end direction. A distal portion of the second rib 40 has a surface intersecting the axial direction of the cap 16 (direction of the arrows A and B).

The lock portion 18 includes a coupling member 42, a first coupling structure 44, and a second coupling structure 46. When a coupling state of at least one of the first coupling structure 44 and the second coupling structure 46 is released, the lock portion 18 enters an unlocked state in which the cap 16 can be detached from the housing 12.

The coupling member 42 is operated in a direction different from the axial direction of the housing 12 to bring the lock portion 18 into the unlocked state. As shown in Fig. 2, the coupling member 42 is a rotary member 42a that is formed in a hollow cylindrical shape and is provided rotatably about the axis of the housing 12. By rotating the rotary member 42a relative to the housing 12, the lock portion 18 enters the unlocked state. That is, the rotation direction of the coupling member 42 is different from the axial direction of the housing 12 (the direction of the arrows A and B).

The coupling member 42 includes a cylindrical body portion 481, a cylindrical proximal portion 482 provided at a proximal end of the cylindrical body portion 481, a lock rib 50 (hereinafter, referred to as a first rib 50), and an engagement groove 58. As shown in Fig. 4, a distal end of the cylindrical body portion 481 is provided at a distal portion of the coupling member 42. The distal end of the cylindrical body portion 481 includes a distal end opening 521 opened in the axial direction. The proximal portion of the cap 16 is inserted into the distal end opening 521. A proximal end of the cylindrical proximal portion 482 is provided at a proximal portion of the coupling member 42. The proximal end of the cylindrical proximal portion 482 includes a proximal end opening 522 opened in the axial direction. The distal portion of the housing 12 is inserted into the proximal end opening 522. In the coupling member 42, an internal space 523 is provided in which the cap 16 and a part of the housing 12 are accommodated. The internal space 523 communicates with the distal end opening 521 and the proximal end opening 522.

As shown in Fig. 2, an outer wall of the cylindrical body portion 481 includes a second indicator 202 and a grip portion 56. The second indicator 202 has a plurality of hole portions 54 penetrating the outer wall radially. In the present embodiment, a case where a pair of the hole portions 54 is provided will be described. The hole portions 54 are not limited to being provided as a pair. For example, the number of the hole portions 54 is equal to the number of the window portions 22 of the first indicator 201. When the coupling member 42 is viewed from the outer peripheral side, for example, the hole portion 54 is formed in a rectangular shape. The present invention is not limited to the case where the first indicator 201 has the window portions 22 and the second indicator 202 has the hole portions 54. For example, the first indicator 201 may be a first mark provided on the housing outer wall 121, and the second indicator 202 may be a second mark provided on the cap outer wall 162.

A plurality of the grip portions 56 are provided apart from each other in the circumferential direction of the cylindrical body portion 481. The grip portion 56 protrudes outward from the outer wall, and extends along the axial direction of the coupling member 42. When the user grips and operates the coupling member 42, the grip portion 56 facilitates the operation of the coupling member 42.

As shown in Fig. 4, the first rib 50 is provided at the distal portion of the cylindrical body portion 481, and protrudes radially inward from the inner wall of the distal portion. The first ribs 50 are provided in the distal-end direction of the pair of hole portions 54 (direction of the arrow B). That is, the number of the first ribs 50 and the number of the hole portions 54 are the same. The number of the first ribs 50 is not limited to being the same as the number of the hole portions 54. For example, the number of the first ribs 50 may be two or more. The first rib 50 may have an annular shape along the circumferential direction of the coupling member 42. An inner surface of the first rib 50 is tapered toward the proximal-end direction (direction of the arrow A). A proximal portion of the first rib 50 has a surface intersecting the axial direction of the coupling member 42 (direction of the arrows A and B).

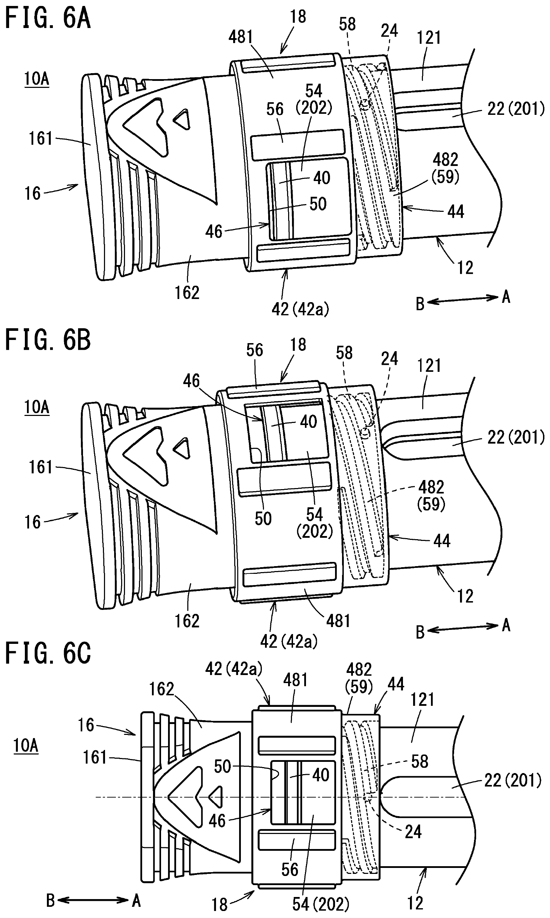

The cylindrical proximal portion 482 extends from the proximal end of the cylindrical body portion 481 in the proximal-end direction (direction of the arrow A). The cylindrical proximal portion 482 is a groove forming portion 59 including an engagement groove 58. As shown in Fig. 6A, the engagement groove 58 extends along the circumferential direction of the cylindrical proximal portion 482. The engagement groove 58 has a spiral shape centered on the axis of the coupling member 42. The engagement protrusion 24 of the housing 12 is engaged with the engagement groove 58. The groove forming portion 59 is transparent. The engagement groove 58 can be visually confirmed from the outside of the coupling member 42 through the groove forming portion 59. In the coupling member 42, at least the cylindrical proximal portion 482 having the groove forming portion 59 may be transparent, and for example, the entire coupling member 42 including the cylindrical body portion 481 may be transparent.

As shown in Fig. 5, the first coupling structure 44 couples the housing 12 and the coupling member 42. The first coupling structure 44 is an engagement structure in which the engagement protrusion 24 of the housing 12 and the engagement groove 58 of the coupling member 42 are engaged with each other. When the distal portion of the housing 12 and the coupling member 42 are coupled by the lock portion 18, the distal portion of the housing 12 is inserted into the internal space 523 of the coupling member 42 and the engagement protrusion 24 is inserted into the engagement groove 58 for mutual engagement. Due to the first coupling structure 44, a coupling state is reached in which the movement of the coupling member 42 in the distal-end direction (axial direction) relative to the distal portion of the housing 12 is prevented.

As shown in Fig. 6B, by rotating the coupling member 42 relative to the housing 12 about the axis of the housing 12, the engagement protrusion 24 can move along the engagement groove 58. As the coupling member 42 rotates, the coupling member 42 is movable relative to the housing 12 in the distal-end direction. The engagement between the engagement protrusion 24 and the engagement groove 58 is released, the coupling member 42 moves in the distal-end direction (the direction of the arrow B), and the engagement protrusion 24 of the housing 12 disengages from the engagement groove 58 of the coupling member 42. By moving the cap 16 relative to the distal portion of the housing 12 in the distal-end direction (the direction of the arrow B), a first coupling released state is reached in which the coupling state between the housing 12 and the coupling member 42 by the first coupling structure 44 is released. In the first coupling released state, an unlocked state in which the cap 16 can be detached from the distal end of the housing 12 is achieved. For example, the engagement groove 58 is formed so that the engagement with the engagement protrusion 24 can be released when the coupling member 42 is rotated about 90° about the central axis of the housing 12. The engagement groove 58 may be formed so that the engagement with the engagement protrusion 24 is released when the coupling member 42 is rotated 90° or more about the central axis of the housing 12. The engagement groove 58 may be formed so that the engagement with the engagement protrusion 24 is released when the coupling member 42 is rotated less than 90° about the central axis of the housing 12.

The engagement groove 58 constituting the first coupling structure 44 is not limited to being formed in a spiral shape. For example, the engagement groove 58 may have a circumferential groove portion extending in the circumferential direction of the cylindrical proximal portion 482, and an axial groove portion extending from an end portion of the circumferential groove portion toward the proximal end of the cylindrical proximal portion 482. In this case, the housing 12 and the coupling member 42 are coupled in the axial direction by engaging the engagement protrusion 24 with the circumferential groove portion. By rotating the coupling member 42 so that the engagement protrusion 24 is moved and disengaged along the axial groove portion, the coupling between the coupling member 42 and the housing 12 can be released.

As shown in Fig. 4, the second coupling structure 46 couples the cap 16 and the coupling member 42. When the proximal portion of the cap 16 is accommodated in the internal space 523 of the coupling member 42, the proximal portion of the first rib 50 faces the distal portion of the second rib 40 in the axial direction of the housing 12 (see Fig. 6C). The second coupling structure 46 is an abutment structure in which the proximal portion of the first rib 50 and the distal portion of the second rib 40 can abut on each other when the cap 16 and the coupling member 42 relatively move in the axial direction.

Next, the use of the medicinal liquid administration device 10A will be described. In the medicinal liquid administration device 10A before use shown in Fig. 1, since the housing 12 and the cap 16 are coupled in the axial direction by the coupling member 42 of the lock portion 18, the cap 16 is not erroneously detached from the distal portion of the housing 12.

First, the cap 16 is detached from the medicinal liquid administration device 10A before use. Specifically, the coupling between the housing 12 and the coupling member 42 by the first coupling structure 44 of the lock portion 18 is released.

In the locked state of the lock portion 18 shown in Fig. 6A, the housing 12 is in a fixed state, and the coupling member 42 is rotated about the axis of the housing 12. As shown in Fig. 6B, as the coupling member 42 rotates, the engagement protrusion 24 moves along the engagement groove 58, and thus the coupling member 42 moves relative to the housing 12 in the distal-end direction (direction of the arrow B). Due to the movement of the coupling member 42 in the distal-end direction, the proximal portion of the first rib 50 and the distal portion of the second rib 40 in the second coupling structure 46 are separated from each other in the axial direction.

When the engagement protrusion 24 of the housing 12 disengages from the engagement groove 58 of the coupling member 42, the engagement state between the engagement groove 58 and the engagement protrusion 24 is released, resulting in a first coupling released state in which the coupling member 42 is movable relative to the distal portion of the housing 12 in the distal-end direction. In this case, the user can visually confirm that the engagement state between the engagement groove 58 and the engagement protrusion 24 has been released from the outside of the coupling member 42 through the transparent groove forming portion 59. That is, the first coupling released state is reached in which the coupling state of the first coupling structure 44 is released.

In the unlocked state, as shown in Fig. 6C, the first indicator 201 (window portion 22) and the second indicator 202 (hole portion 54) are linearly disposed in the axial direction of the housing 12 and the coupling member 42. When the first and second indicators 201 and 202 are linearly disposed, it is also possible to visually confirm that the first coupling released state has been reached.

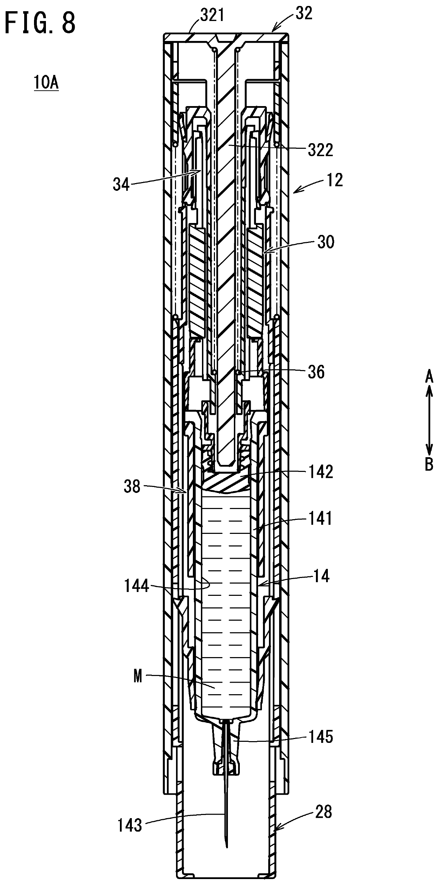

In the first coupling released state, the user grips the cap 16 and pulls the cap 16 in a direction away from the housing 12 (distal-end direction, direction of the arrow B). Therefore, the cap 16 moves relative to the distal portion of the housing 12 in the distal-end direction, and the holding arm 163 shown in Fig. 3 tilts, causing the hook 164 to disengage from the recess portion 166. The engagement of the holding arm 163 (hook 164) with the needle cover 28 is released. As shown in Fig. 7, the cap 16 is detached from the distal end of the housing 12 in the distal-end direction. Therefore, the distal ends of the housing 12 and the needle cover 28 are open, and at the same time, a protective cover 60 that has been covering the puncture needle 143 is detached (See Fig. 8).

In this case, by pulling the cap 16 in the distal-end direction, the distal portion of the second rib 40 abuts on the proximal portion of the first rib 50, and the coupling member 42 is pulled in the distal-end direction together with the cap 16. Since the coupling member 42 and the cap 16 are in a coupling state by the second coupling structure 46, the coupling member 42 is detached from the housing 12 together with the cap 16. For example, the cap 16 and the coupling member 42 are discarded without being reused.

Next, administration of the medicinal liquid M using the medicinal liquid administration device 10A from which the cap 16 is detached will be described.

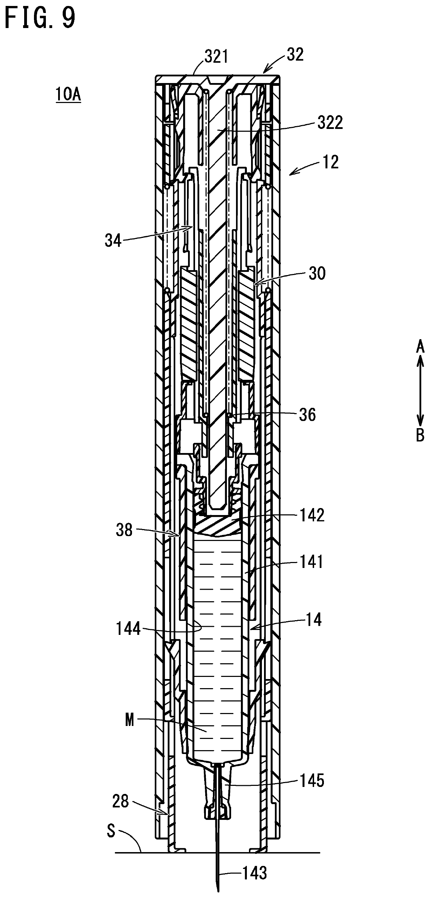

As shown in Fig. 8, in the medicinal liquid administration device 10A, the distal portion of the needle cover 28 protrudes from the distal end of the housing 12 in the distal-end direction (direction of the arrow B) in a state in which the cap 16 is detached. As shown in Fig. 9, the distal end of the needle cover 28 is pressed against a skin S, which is a desired target for puncture in the living body, at approximately a right angle, and the housing 12 is further continuously pushed toward the skin S (distal-end direction, direction of the arrow B). With the housing 12 being pushed toward the skin S, the needle cover 28 is pressed against the skin S and moves relative to the housing 12 in the proximal-end direction (direction of the arrow A).

With the housing 12 being further pushed toward the skin S, the puncture needle 143 of the syringe 14 protrudes from a hole portion of the needle cover 28 in the distal-end direction (direction of the arrow B). Therefore, a puncture completed state is reached in which the puncture needle 143 punctures the skin S and is inserted to a predetermined depth. In the puncture completed state, the distal end of the housing 12 is substantially at the same position as the distal end of the needle cover 28.

The locked state of the plunger 34 by a lock mechanism (not shown) is released, the plunger 34 starts to move in the distal-end direction (direction of the arrow B) by the resilient force of the injection spring 36, and the stopper 142 is inserted into the cylindrical body 141 of the syringe 14 by the movement of the plunger 34.

After the administration of the medicinal liquid M is started, the plunger 34 continuously moves in the distal-end direction (direction of the arrow B) at a constant speed by the resilient force of the injection spring 36. The medicinal liquid M is pushed out by the stopper 142 moving in the distal-end direction inside the cylindrical body 141, and the medicinal liquid M is discharged from the puncture needle 143. The plunger 34 administers a predetermined amount of the medicinal liquid M in the cylindrical body 141 to the skin S through the puncture needle 143, and the administration of the medicinal liquid M is completed.

The first embodiment has the following effects.

As shown in Fig. 4, the medicinal liquid administration device 10A includes the lock portion 18 that couples the cap 16 and the housing 12, and the lock portion 18 includes the first coupling structure 44 that couples the housing 12 and the coupling member 42, and the second coupling structure 46 that couples the cap 16 and the coupling member 42. As shown in Fig. 6B, when the coupling state of at least one (first coupling structure 44) of the first coupling structure 44 and the second coupling structure 46 is released, an unlocked state is reached in which the cap 16 can be detached from the housing 12.

Therefore, in a state in which the locked state by the first coupling structure 44 or the second coupling structure 46 of the lock portion 18 is not released, the cap 16 is prevented from being detached from the distal end of the housing 12. Accordingly, it is possible to reduce the occurrence of erroneous puncture due to erroneous detachment of the cap 16 from the distal end of the housing 12.

As shown in Fig. 6A, the coupling member 42 is operated in a direction different from the axial direction of the housing 12, and thus the lock portion 18 enters the unlocked state. Therefore, since the operation direction of the coupling member 42 is different from the axial direction of the housing 12, the coupling member 42 is less likely to be erroneously operated by the user. That is, the lock portion 18 is a lock capable of preventing the cap 16 from being erroneously detached from the housing 12 by the user in the medicinal liquid administration device 10A before use.

As shown in Fig. 6B, by releasing the coupling state between the housing 12 and the coupling member 42 by the first coupling structure 44, an unlocked state can be reached in which the cap 16 can be detached from the distal end of the housing 12 as shown in Fig. 7. When the cap 16 is detached from the housing 12, the coupling member 42 is detached from the housing 12 together with the cap 16 in a state in which the cap 16 and the coupling member 42 are coupled by the second coupling structure 46. Therefore, when the coupling state by the first coupling structure 44 is released and the cap 16 is detached from the distal end of the housing 12, the coupling member 42 can be detached together with the cap 16. Accordingly, the second coupling structure 46 does not remain on the housing 12, and the coupling member 42 can be discarded together with the cap 16.

As shown in Fig. 6A, the coupling member 42 is a rotary member 42a that is formed in a cylindrical shape and is provided rotatably about the axis of the housing 12, and enters an unlocked state by rotating the rotary member 42a. Therefore, the coupling member 42 can be constituted with a simple configuration, and thus the cost can be reduced.

As shown in Fig. 4, the coupling member 42 has the first rib 50 that protrudes from the inner wall of the distal portion having the distal end opening 521, and the engagement groove 58 that is provided on the inner wall of the proximal portion having the proximal end opening 522 and extends in the circumferential direction. As shown in Fig. 5, the cap 16 has the second rib 40 that is provided at the proximal portion and protrudes from the cap outer wall 162, and the distal portion of the housing 12 has the engagement protrusion 24 protruding from the housing outer wall 121. The first coupling structure 44 is an engagement structure in which the engagement protrusion 24 and the engagement groove 58 are engaged with each other, and the second coupling structure 46 is an abutment structure in which the proximal portion of the first rib 50 and the distal portion of the second rib 40 face each other in the axial direction of the housing 12 and the proximal portion and the distal portion can abut on each other. As shown in Fig. 6B, by rotating the coupling member 42 relative to the housing 12, the engagement protrusion 24 disengages from the engagement groove 58, and the coupling state of the first coupling structure 44 is released.

Therefore, the first coupling structure 44 can effectively couple the housing 12 and the coupling member 42 by engaging the engagement protrusion 24 with the engagement groove 58. The coupling by the first coupling structure 44 can be easily released by rotating the coupling member 42 relative to the housing 12. By causing the first rib 50 and the second rib 40 to abut on each other in the axial direction when the cap 16 is detached from the housing 12, the cap 16 is prevented from being moved in the distal-end direction relative to the coupling member 42, and the coupling member 42 can be detached together with the cap 16.

As shown in Fig. 6A, the engagement groove 58 of the coupling member 42 has a spiral shape centered on the axis of the coupling member 42, and as shown in Fig. 6B, when the coupling state of the first coupling structure 44 is released, the engagement protrusion 24 moves along the engagement groove 58, causing the coupling member 42 to move in the distal-end direction relative to the housing 12. When the cap 16 is detached from the housing 12 in the distal-end direction, the proximal portion of the first rib 50 and the distal portion of the second rib 40 abut on each other. Therefore, the coupling state of the first coupling structure 44 can be easily released by rotating the coupling member 42 relative to the housing 12. When the cap 16 is detached from the housing 12, the coupling member 42 can be easily detached together with the cap 16 by the second coupling structure 46.

As shown in Fig. 6C, the housing 12 has the first indicator 201, and the coupling member 42 has the second indicator 202. By rotating the coupling member 42 relative to the housing 12, the first indicator 201 and the second indicator 202 are linearly disposed in the axial direction of the housing 12 and the coupling member 42, and a first coupling released state is reached in which the coupling by the first coupling structure 44 is released. Therefore, when the coupling member 42 is rotated relative to the housing 12, the user can easily visually confirm that the rotation of the coupling member 42 has been completed and the coupling of the first coupling structure 44 has been released through the first and second indicators 201 and 202.

As shown in Fig. 1, the first indicator 201 is the window portion 22 through which the syringe 14 can be visually confirmed from the outside of the housing 12, and the second indicator 202 is the hole portion 54 penetrating the outer wall of the coupling member 42. Therefore, by using the window portion 22 provided for confirming the syringe 14, the manufacturing cost of the medicinal liquid administration device 10A can be reduced compared to a structure in which a new first indicator 201 is provided. By providing the second indicator 202 as the hole portion 54, the second indicator 202 can be easily provided in the coupling member 42.

As shown in Fig. 6B, the coupling member 42 has the groove forming portion 59 including the engagement groove 58, and the groove forming portion 59 is transparent. Therefore, since the engagement state between the engagement protrusion 24 and the engagement groove 58 can be confirmed from the outside of the coupling member 42 through the transparent groove forming portion 59, the user can effectively confirm that the rotation of the coupling member 42 has been completed.

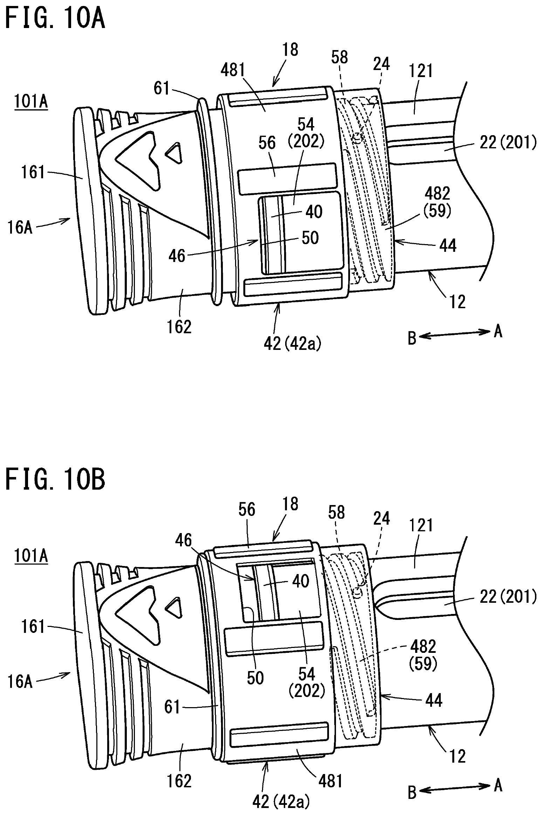

As shown in Fig. 10A, a medicinal liquid administration device 101A according to a modification of the first embodiment has a cap 16A, and a cap outer wall 162 of the cap 16A has an abutment portion 61. The abutment portion 61 can abut on the distal portion of the coupling member 42. The abutment portion 61 is provided at a proximal portion of the cap 16A. The abutment portion 61 is formed in an annular shape and has a rib shape protruding radially outward from the cap outer wall 162. The abutment portion 61 is orthogonal to the axial direction of the cap 16A. The abutment portion 61 is not limited to being formed in an annular shape. For example, the abutment portion 61 may have a protruding shape provided on a part of the cap 16A in the circumferential direction. In the coupling state (locked state) between the housing 12 and the cap 16A by the lock portion 18, the distal portion of the coupling member 42 and the abutment portion 61 are disposed to be separated from each other in the axial direction of the housing 12.

When the cap 16A is detached from the housing 12, the coupling member 42 is rotated relative to the housing 12 to be moved in the distal-end direction, and as shown in Fig. 10B, the distal portion of the coupling member 42 and the abutment portion 61 abut on each other when the engagement protrusion 24 disengages from the engagement groove 58. The abutment portion 61 prevents the movement of the coupling member 42 in the distal-end direction. When the locked state by the first coupling structure 44 is released due to the disengagement of the engagement protrusion 24 from the engagement groove 58 and a first coupling released state is reached, the distal portion of the coupling member 42 and the abutment portion 61 abut on each other.

The modification of the first embodiment has the following effects.

Since the abutment portion 61 is provided on the cap outer wall 162 of the cap 16A, when the coupling between the housing 12 and the coupling member 42 by the first coupling structure 44 is released, a user can effectively confirm that the rotation of the coupling member 42 has been completed and the first coupling released state has been reached in which the coupling by the first coupling structure 44 is released, by recognizing that the coupling member 42 has moved in the distal-end direction and its distal portion has abutted on the abutment portion 61. Accordingly, even in a case where the groove forming portion 59 of the coupling member 42 is not transparent, the user can recognize that the coupling state by the first coupling structure 44 has been released.

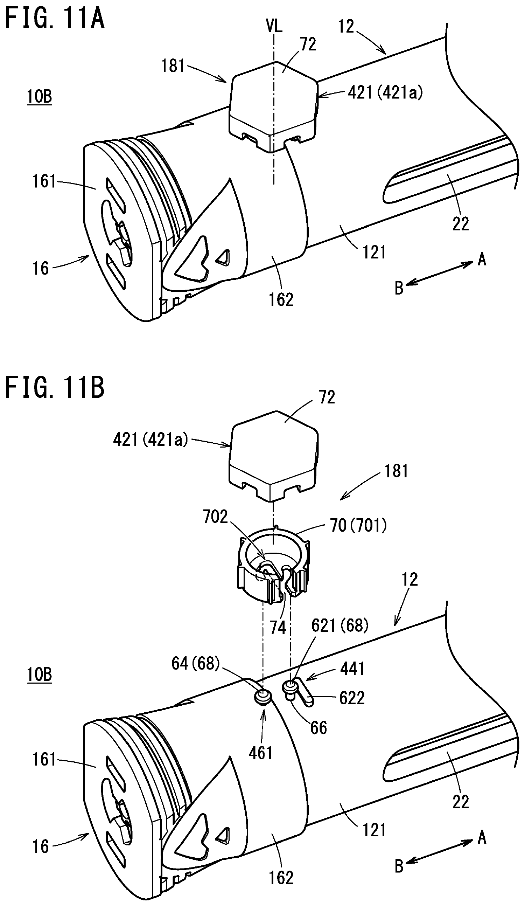

As shown in Fig. 11A, a medicinal liquid administration device 10B according to a second embodiment includes a lock portion 181 that couples a cap 16 and a housing 12. As shown in Fig. 11B, the lock portion 181 has a coupling member 421, a first coupling structure 441 that couples the housing 12 and the coupling member 421, and a second coupling structure 461 that couples the cap 16 and the coupling member 421.

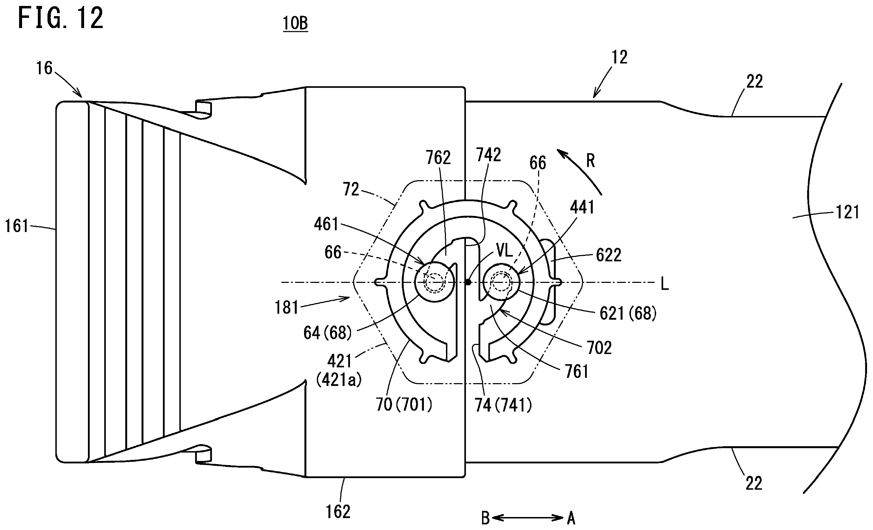

As shown in Fig. 12, in a locked state in which the housing 12 and the cap 16 are coupled by the first and second coupling structures 441 and 461, the coupling member 421 is provided on a cap outer wall 162 and disposed across a proximal portion of the cap 16 and a distal portion of the housing 12. The coupling member 421 is a rotary member 421a that is rotatable about a vertical axis VL perpendicular to the axial direction of the housing 12. By rotating the rotary member 421a, an unlocked state is reached in which the cap 16 can be detached from the distal end of the housing 12. That is, the coupling member 421 is operated in a rotation direction that is a direction different from the axial direction of the housing 12 to bring the lock portion 181 into the unlocked state.

As shown in Fig. 11B, the distal portion of the housing 12 has a first engagement protrusion 621 protruding from a housing outer wall 121 and a pedestal portion 622. The pedestal portion 622 is disposed in the proximal-end direction of the first engagement protrusion 621 (direction of the arrow A). The pedestal portion 622 protrudes from the housing outer wall 121. The pedestal portion 622 extends in a direction orthogonal to the axial direction of the housing 12. The pedestal portion 622 has a flat distal end surface.

The cap 16 has a second engagement protrusion 64 protruding from the cap outer wall 162. In a state in which the housing 12 and the cap 16 are coupled by the lock portion 181, the first engagement protrusion 621 and the second engagement protrusion 64 are linearly disposed in the axial direction of the housing 12. Each of the first and second engagement protrusions 621 and 64 has a pin portion 66 and a head portion 68 formed at a distal end of the pin portion 66 and having a larger diameter than the pin portion 66. The protruding direction of the first and second engagement protrusions 621 and 64 is perpendicular to the axial direction of the housing 12.

The rotary member 421a includes an inner member 70 and an outer cover 72. The inner member 70 includes a body portion 701 and an engagement groove 702. The body portion 701 is formed in a circular shape in plan view of the rotary member 421a. A part of the body portion 701 is supported by abutting on a surface of the pedestal portion 622.

As shown in Fig. 12, the engagement groove 702 has a linear groove 74, a first engagement groove 761, and a second engagement groove 762. The linear groove 74 is formed in a linear shape passing through the center of the body portion 701 in plan view of the rotary member 421a. The linear groove 74 has an open end 741 and a closed end 742. The open end 741 is formed at one end of the linear groove 74 and opens on an outer circumferential surface of the body portion 701. The closed end 742 is formed at the other end of the linear groove 74 and is disposed inside the outer circumferential surface of the body portion 701.

The first and second engagement grooves 761 and 762 are formed in an arc shape along the outer circumferential surface of the body portion 701. The first engagement groove 761 and the second engagement groove 762 are disposed on opposite sides with the linear groove 74 interposed therebetween.

The first engagement groove 761 engages with the first engagement protrusion 621. The pin portion 66 of the first engagement protrusion 621 is movably inserted into the first engagement groove 761. The first engagement groove 761 has an arc shape extending along the rotation direction of the rotary member 421a about the vertical axis VL. The first engagement groove 761 is coupled to a substantially central portion in the extending direction of the linear groove 74. The first engagement groove 761 and the linear groove 74 intersect at a coupling part. The first engagement groove 761 extends in an arc shape from the coupling part with the linear groove 74. In a locked state by the lock portion 181, the first engagement groove 761 is disposed to face the housing outer wall 121 of the housing 12.

The second engagement groove 762 engages with the second engagement protrusion 64. The pin portion 66 of the second engagement protrusion 64 is movably inserted into the second engagement groove 762. The second engagement groove 762 has an arc shape extending along the rotation direction of the rotary member 421a about the vertical axis VL. The second engagement groove 762 is coupled to the vicinity of the closed end 742 of the linear groove 74. The second engagement groove 762 and the linear groove 74 intersect at a coupling part. The second engagement groove 762 extends in an arc shape from the coupling part with the linear groove 74. In a locked state by the lock portion 181, the second engagement groove 762 is disposed to face the cap outer wall 162 of the cap 16. The end portions of the first and second engagement grooves 761 and 762 extend to an imaginary line L orthogonal to the extending direction of the linear groove 74. Due to the first and second coupling structures 441 and 461, a locked state is reached in which the relative movement between the housing 12 and the cap 16 in the axial direction is prevented.

The outer cover 72 is gripped when a user operates the coupling member 421. The outer cover 72 covers the inner member 70. The outer cover 72 is formed in a regular hexagonal shape in plan view. The shape of the outer cover 72 is not limited to a regular hexagonal shape. The shape is not particularly limited as long as it is a support shape that can be gripped by the user and is rotatable about the vertical axis VL. The inner member 70 is accommodated inside the outer cover 72. When the inner member 70 engages with the outer cover 72, the inner member 70 and the outer cover 72 are integrally rotatable.

The first coupling structure 441 is an engagement structure between the first engagement protrusion 621 of the housing 12 and the first engagement groove 761 of the coupling member 421. The second coupling structure 461 is a fixing structure between the coupling member 421 and the cap 16.

When the user operates the coupling member 421 so that the first engagement protrusion 621 moves along the first engagement groove 761 and the second engagement protrusion 64 moves along the second engagement groove 762, the coupling member 421 rotates about the vertical axis VL perpendicular to the axial direction of the housing 12.

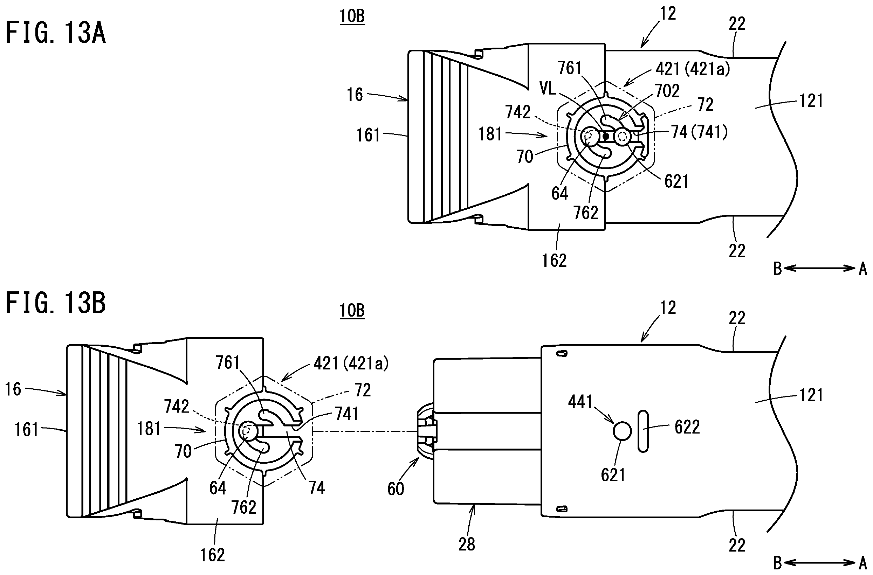

When the coupling state between the housing 12 and the coupling member 421 by the first coupling structure 441 of the lock portion 181 is released, the user grips and rotates the coupling member 421 in a first direction R about the vertical axis VL from the locked state of the lock portion 181 shown in Fig. 12. As shown in Fig. 13A, due to the rotation of the coupling member 421, the first engagement protrusion 621 moves toward the linear groove 74 along the first engagement groove 761, and the second engagement protrusion 64 moves toward the linear groove 74 along the second engagement groove 762. When the first engagement protrusion 621 disengages from the first engagement groove 761 and reaches the linear groove 74, a first coupling released state is reached in which the coupling state by the first coupling structure 441 is released. The second engagement protrusion 64 disengages from the second engagement groove 762 and reaches the vicinity of the closed end 742 of the linear groove 74.

When the user grips and pulls the cap 16 in the distal-end direction (direction of the arrow B) relative to the housing 12 as shown in Fig. 13B, the first engagement protrusion 621 moves along the linear groove 74 and disengages from the linear groove 74 through the open end 741. Therefore, the coupling member 421 is separated from the housing 12. By pulling the cap 16 in the distal-end direction relative to the housing 12, the coupling member 421 is pulled in the distal-end direction together with the cap 16 in a state in which the second engagement protrusion 64 is caught on the closed end 742 of the second engagement groove 762. That is, the coupling member 421 is detached from the housing 12 together with the cap 16 while the coupling state between the cap 16 and the coupling member 421 by the second coupling structure 461 is maintained.

The second embodiment has the following effects.

As shown in Fig. 12, the coupling member 421 is the rotary member 421a that is provided across the proximal portion of the cap 16 and the distal portion of the housing 12 and is rotatable about the vertical axis VL perpendicular to the axial direction of the housing 12. As shown in Fig. 13A, by rotating the rotary member 421a, an unlocked state is reached in which the cap 16 can be detached from the housing 12. Therefore, by providing the coupling member 421 as the rotary member 421a that is rotatable about the vertical axis VL perpendicular to the axial direction of the housing 12, the coupling member 421 can be constituted with a simple configuration, and the cost can be reduced.

As shown in Fig. 11B, the distal portion of the housing 12 has the first engagement protrusion 621 protruding from the housing outer wall 121, the coupling member 421 has the first engagement groove 761 with which the first engagement protrusion 621 engages, and the first engagement groove 761 extends along the rotation direction about the vertical axis VL. The first coupling structure 441 is an engagement structure between the first engagement protrusion 621 and the first engagement groove 761, and the second coupling structure 461 is a fixing structure between the coupling member 421 and the cap 16.

Therefore, as shown in Fig. 13B, when the coupling state by the first coupling structure 441 is released and the cap 16 is detached, the coupling member 421 coupled to the cap 16 by the second coupling structure 461 can be detached together. Therefore, the coupling member 421 can be discarded together with the cap 16 without remaining on the housing 12. The coupling between the housing 12 and the coupling member 421 by the first coupling structure 441 can be easily released by rotating the coupling member 421 about the vertical axis VL.

As shown in Fig. 11B, the cap 16 has the second engagement protrusion 64 protruding from the cap outer wall 162, and the coupling member 421 has the second engagement groove 762 with which the second engagement protrusion 64 engages. As shown in Fig. 13A, by rotating the coupling member 421 about the vertical axis VL, the second engagement protrusion 64 moves along the second engagement groove 762.

Therefore, when the coupling member 421 is rotated about the vertical axis VL, the rotation of the coupling member 421 is effectively stabilized due to the movement of the second engagement protrusion 64 along the second engagement groove 762.

The coupling member 421 is operated in a rotation direction about the vertical axis VL that is different from the axial direction of the housing 12, and thus the lock portion 181 enters the unlocked state. Therefore, since the operation direction of the coupling member 421 is different from the axial direction of the housing 12, the coupling member 421 is less likely to be erroneously operated by the user.

In a case where the lock portion 181 is brought into the unlocked state, only the coupling state of the first coupling structure 441 may be released, and the coupling member 421 may not be detached together with the cap 16. For example, as shown in Fig. 13B, after the first coupling released state is reached in which the coupling state of the first coupling structure 441 is released, the second engagement protrusion 64 may be disengaged from the linear groove 74 to reach a second coupling released state in which the coupling between the coupling member 421 and the cap 16 by the second coupling structure 461 is released.

Therefore, as shown in Fig. 13B, when the cap 16 is detached from the housing 12, the coupling member 421 can be detached from the cap 16 by further moving the coupling member 421 relative to the cap 16 in the distal-end direction (direction of the arrow B). That is, each of the coupling states by the first and second coupling structures 441 and 461 is released, and the coupling member 421 can be detached from both the housing 12 and the cap 16.

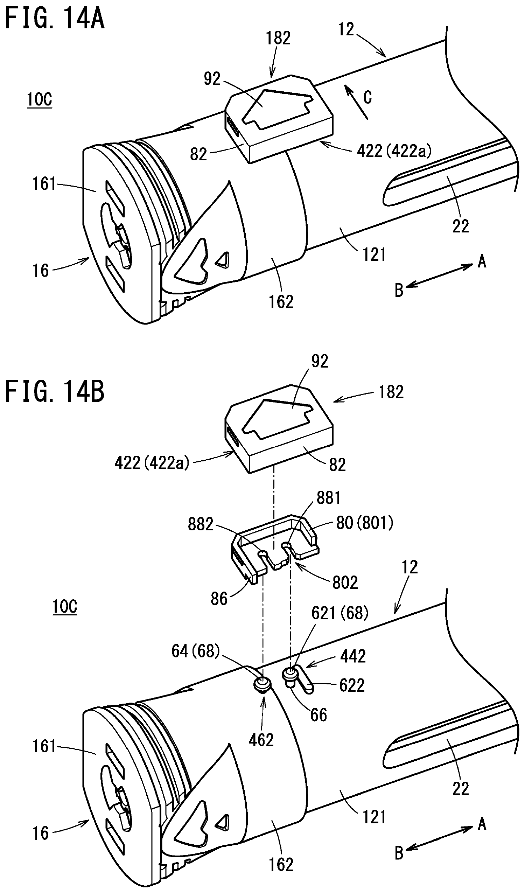

As shown in Fig. 14A, a medicinal liquid administration device 10C according to a third embodiment includes a lock portion 182 that couples a cap 16 and a housing 12. As shown in Fig. 14B, the lock portion 182 has a coupling member 422, a first coupling structure 442 that couples the housing 12 and the coupling member 422, and a second coupling structure 462 that couples the cap 16 and the coupling member 422.

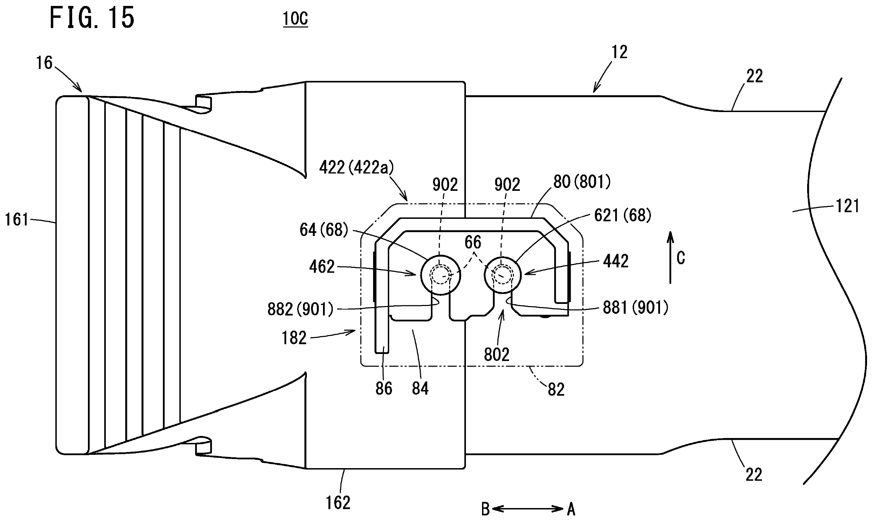

As shown in Fig. 15, in a locked state in which the housing 12 and the cap 16 are coupled by the first and second coupling structures 442 and 462, the coupling member 422 is disposed across a proximal portion of the cap 16 and a distal portion of the housing 12. The coupling member 422 is a slide member 422a that is slidable in an intersecting direction (direction of the arrow C in Fig. 15) intersecting the axial direction of the housing 12 (direction of the arrows A and B). By moving the slide member 422a in the intersecting direction (direction of the arrow C), an unlocked state is reached in which the cap 16 can be detached from the housing 12. That is, the coupling member 422 is operated in the intersecting direction (direction of the arrow C) that is a direction different from the axial direction of the housing 12 to bring the lock portion 182 into the unlocked state. In the following description, a configuration will be described in which the coupling member 422 is movable in a direction orthogonal to the axial direction of the housing 12.

As shown in Fig. 14B, the distal portion of the housing 12 has a first engagement protrusion 621 protruding from a housing outer wall 121. The cap 16 has a second engagement protrusion 64 protruding from a cap outer wall 162.

A slide member 422a includes an inner member 80 and an outer cover 82. The inner member 80 includes a body portion 801 and an engagement groove 802. As shown in Fig. 15, the body portion 801 is formed in a rectangular shape that is long in the axial direction of the housing 12 in plan view of the slide member 422a. The body portion 801 is not limited to being formed in a rectangular shape.

As shown in Fig. 15, the body portion 801 includes an accommodation portion 84 and a locking wall 86. The accommodation portion 84 is provided at one end of the slide member 422a in the moving direction. The accommodation portion 84 has a space provided on one side in a direction intersecting the axial direction of the housing 12. The locking wall 86 is disposed in the distal-end direction, facing the accommodation portion 84 in the axial direction of the housing 12. The locking wall 86 protrudes to one side in a direction intersecting the axial direction of the housing 12. In a state in which the cap 16 and the housing 12 are coupled, the locking wall 86 faces the cap outer wall 162 of the cap 16 and is disposed further in the distal-end direction than the second engagement protrusion 64.

The engagement groove 802 has a first engagement groove 881 and a second engagement groove 882. Each of the first and second engagement grooves 881 and 882 extends in a direction orthogonal to the axial direction of the housing 12. That is, each of the first and second engagement grooves 881 and 882 extends along the moving direction of the coupling member 422.

Each of the first and second engagement grooves 881 and 882 has an open end 901 and a closed end 902. The open end 901 is one end in the extending direction of each of the first and second engagement grooves 881 and 882. The open end 901 opens to face the accommodation portion 84. The closed end 902 is the other end in the extending direction of each of the first and second engagement grooves 881 and 882. Each of the first and second engagement grooves 881 and 882 is formed linearly along the moving direction of the coupling member 422. The first engagement groove 881 and the second engagement groove 882 are parallel to each other. In a locked state by the lock portion 182, the first engagement groove 881 is disposed to face the housing outer wall 121 of the housing 12, and the second engagement groove 882 is disposed to face the cap outer wall 162 of the cap 16. In the moving direction of the coupling member 422, the second engagement groove 882 has a longer length than the first engagement groove 881. That is, the open end 901 of the second engagement groove 882 is disposed closer to the accommodation portion 84 than the open end 901 of the first engagement groove 881.

The first engagement groove 881 engages with the first engagement protrusion 621. A pin portion 66 of the first engagement protrusion 621 is movably inserted into the first engagement groove 881. The second engagement groove 882 engages with the second engagement protrusion 64. A pin portion 66 of the second engagement protrusion 64 is movably inserted into the second engagement groove 882. As shown in Fig. 15, in a locked state by the lock portion 182, the first engagement protrusion 621 is disposed at the closed end 902 of the first engagement groove 881, and the second engagement protrusion 64 is disposed at the closed end 902 of the second engagement groove 882. Due to the first and second coupling structures 442 and 462, a locked state is reached in which the relative movement between the housing 12 and the cap 16 in the axial direction is prevented.

The outer cover 82 is gripped when a user operates the coupling member 422. The outer cover 82 covers the inner member 80. The inner member 80 is accommodated and held inside the outer cover 82. A mark 92 indicating an operation direction of the coupling member 422 is provided on a surface of the outer cover 82. The mark 92 is, for example, an arrow. The direction indicated by the mark 92 is orthogonal to the axial direction of the housing 12. The present invention is not limited to the case where the coupling member 422 includes the mark 92.

The first coupling structure 442 is an engagement structure between the first engagement protrusion 621 of the housing 12 and the first engagement groove 881 of the coupling member 422. The second coupling structure 462 is a fixing structure between the coupling member 422 and the cap 16.

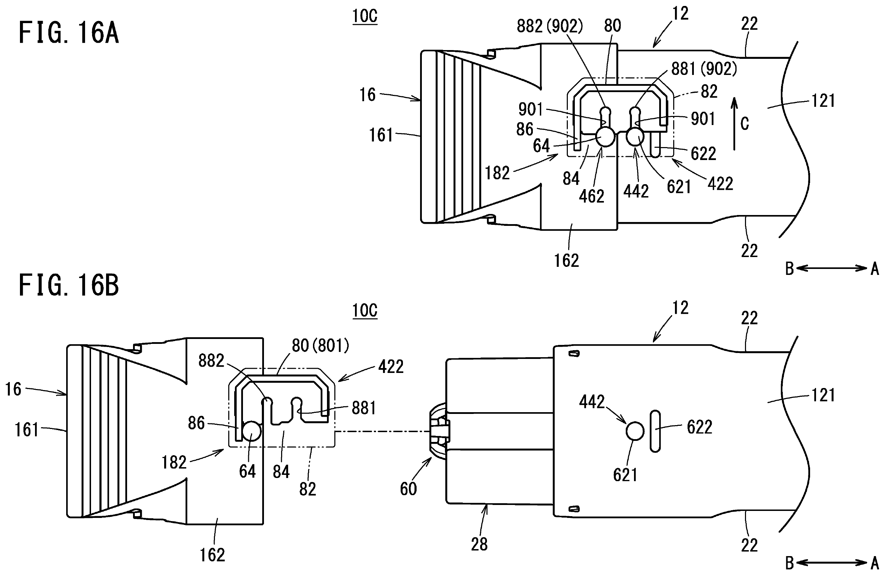

When the coupling state between the housing 12 and the coupling member 422 by the first coupling structure 442 of the lock portion 182 is released, the user slides the coupling member 422 in the intersecting direction (direction of the arrow C) orthogonal to the axial direction of the housing 12 as shown in Fig. 16A. The intersecting direction (direction of the arrow C) is a direction indicated by the mark 92 of the outer cover 82. By sliding the coupling member 422, the first engagement protrusion 621 moves toward the open end 901 along the first engagement groove 881, and the second engagement protrusion 64 moves toward the open end 901 along the second engagement groove 882. When the first engagement protrusion 621 disengages from the first engagement groove 881 and reaches the accommodation portion 84, a first coupling released state is reached in which the coupling state by the first coupling structure 442 is released. In this case, the second engagement protrusion 64 remains engaged with the second engagement groove 882.

When the user grips and pulls the cap 16 in the distal-end direction relative to the housing 12 as shown in Fig. 16B, the coupling member 422 is separated from the housing 12 in the distal-end direction. Therefore, an unlocked state (first coupling released state) is reached in which the coupling between the cap 16 and the housing 12 by the first coupling structure 442 is released. When the cap 16 is pulled in the distal-end direction relative to the housing 12, the second engagement protrusion 64 disengages from the open end 901 of the second engagement groove 882 and moves to the accommodation portion 84, and when the second engagement protrusion 64 moves in the distal-end direction relative to the coupling member 422, the second engagement protrusion 64 abuts on the locking wall 86. The second engagement protrusion 64 causes the coupling member 422 to move in the distal-end direction together with the cap 16 through the locking wall 86. Therefore, the coupling member 422 is detached from the housing 12 together with the cap 16 while the coupling state by the second coupling structure 462 is maintained.

The third embodiment has the following effects.

As shown in Fig. 15, the coupling member 422 is the slide member 422a that is provided across the proximal portion of the cap 16 and the distal portion of the housing 12 and is slidable in the intersecting direction (direction of the arrow C) intersecting the axial direction of the housing 12. Therefore, the coupling member 422 can be constituted with a simple configuration, and thus the cost can be reduced.

As shown in Fig. 14B, the distal portion of the housing 12 has the first engagement protrusion 621 protruding from the housing outer wall 121, and as shown in Fig. 15, the coupling member 422 has the first engagement groove 881 that extends along the intersecting direction (direction of the arrow C) and with which the first engagement protrusion 621 engages. The first coupling structure 442 is an engagement structure between the first engagement protrusion 621 and the first engagement groove 881, and the second coupling structure 462 is a fixing structure between the coupling member 422 and the cap 16.

Therefore, as shown in Fig. 16B, when the coupling state by the first coupling structure 442 is released and the cap 16 is detached from the housing 12, the coupling member 422 coupled by the second coupling structure 462 can be detached together with the cap 16. Therefore, the coupling member 422 can be discarded together with the cap 16 without remaining on the housing 12. The coupling between the housing 12 and the coupling member 422 by the first coupling structure 442 can be easily released by sliding the coupling member 422 in the intersecting direction (direction of the arrow C).

As shown in Fig. 14B, the cap 16 has the second engagement protrusion 64 protruding from the cap outer wall 162, and as shown in Fig. 15, the coupling member 422 has the second engagement groove 882 that extends along the intersecting direction (direction of the arrow C) and with which the second engagement protrusion 64 engages. As shown in Fig. 16A, by moving the coupling member 422 in the intersecting direction (direction of the arrow C), the second engagement protrusion 64 moves along the second engagement groove 882.

Therefore, when the coupling member 422 is slid in the intersecting direction (direction of the arrow C), the movement of the coupling member 422 can be stabilized due to the movement of the second engagement protrusion 64 along the second engagement groove 882.

As shown in Fig. 16A, at least a part of the coupling member 422 is operated in a direction different from the axial direction of the housing 12, and thus the lock portion 182 enters the unlocked state. Therefore, since the operation direction of the coupling member 422 is different from the axial direction of the housing 12, the coupling member 422 is less likely to be erroneously operated by the user.

The coupling member 422 is not limited to being detached together with the cap 16 with the release of only the coupling by the first coupling structure 442. For example, both the coupling states by the first coupling structure 442 and the second coupling structure 462 in the lock portion 182 may be released.

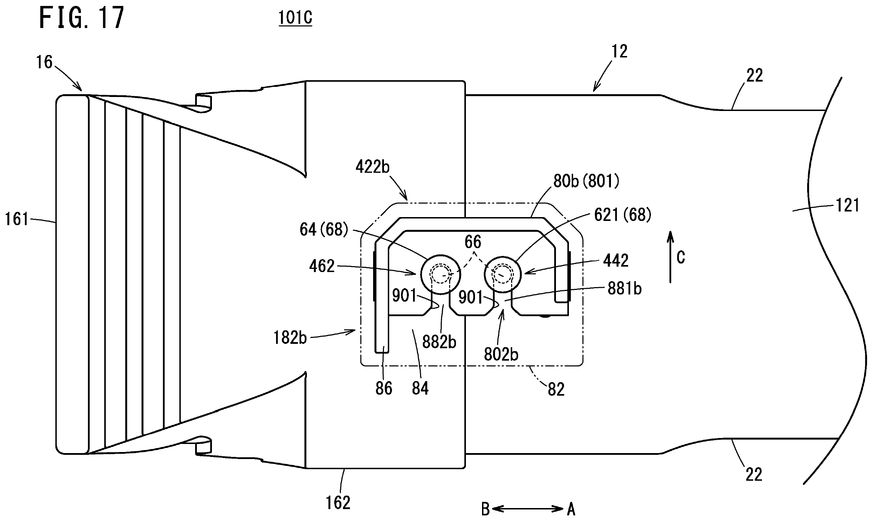

As shown in Fig. 17, in a medicinal liquid administration device 101C according to a modification of the third embodiment, a coupling member 422b of a lock portion 182b includes an inner member 80b. The inner member 80b includes an engagement groove 802b, and the engagement groove 802b has a first engagement groove 881b and a second engagement groove 882b. In the moving direction of the coupling member 422b, the length of the first engagement groove 881b is the same as the length of the second engagement groove 882b.

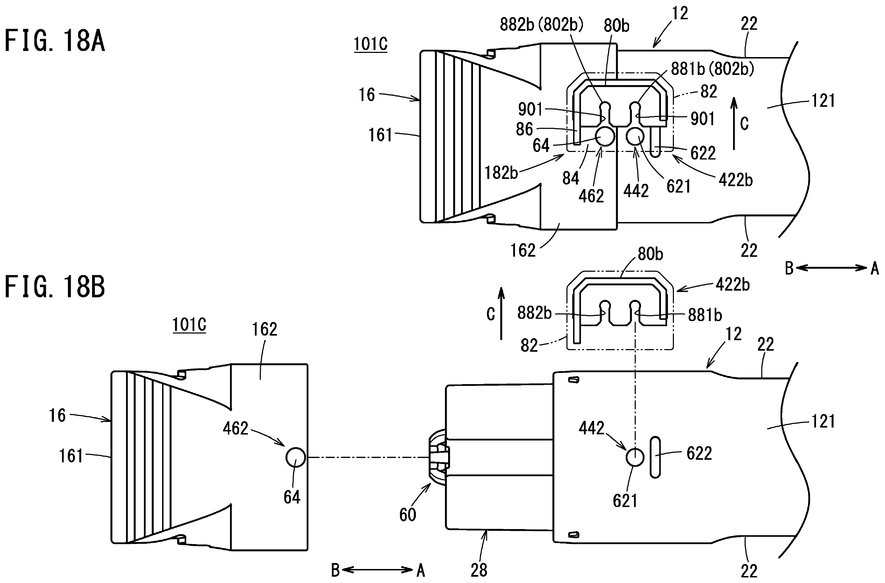

When the coupling state between the housing 12 and the coupling member 422b by the lock portion 182b is released, as shown in Fig. 18A, by sliding the coupling member 422b in the intersecting direction (direction of the arrow C), the first engagement protrusion 621 moves toward the open end 901 along the first engagement groove 881b, and the second engagement protrusion 64 moves toward the open end 901 along the second engagement groove 882b. When the first engagement protrusion 621 disengages from the first engagement groove 881b and reaches the accommodation portion 84, a first coupling released state is reached in which the coupling state by the first coupling structure 442 is released. When the second engagement protrusion 64 disengages from the second engagement groove 882b and reaches the accommodation portion 84, a second coupling released state is reached in which the coupling state by the second coupling structure 462 is released. Since the first and second engagement grooves 881b and 882b have the same length, the coupling states of the first and second coupling structures 442 and 462 are released substantially simultaneously.

As shown in Fig. 18B, when the cap 16 is pulled in the distal-end direction relative to the housing 12 after disengagement of the coupling member 422b from the housing 12 and the cap 16, the cap 16 is detached from the housing 12.

The modification of the third embodiment has the following effects.

When the coupling state by the first coupling structure 442 is released and the cap 16 is detached from the housing 12, the coupling member 422b coupled to the cap 16 by the second coupling structure 462 can be detached together as shown in Fig. 18B. Therefore, the second coupling structure 462 does not remain on the housing 12, and the coupling member 422b can be discarded together with the cap 16. The coupling between the housing 12 and the coupling member 422b by the first coupling structure 442 can be easily released by sliding the coupling member 422b in the intersecting direction.

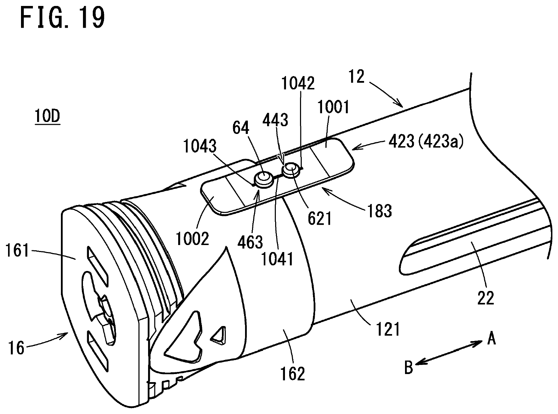

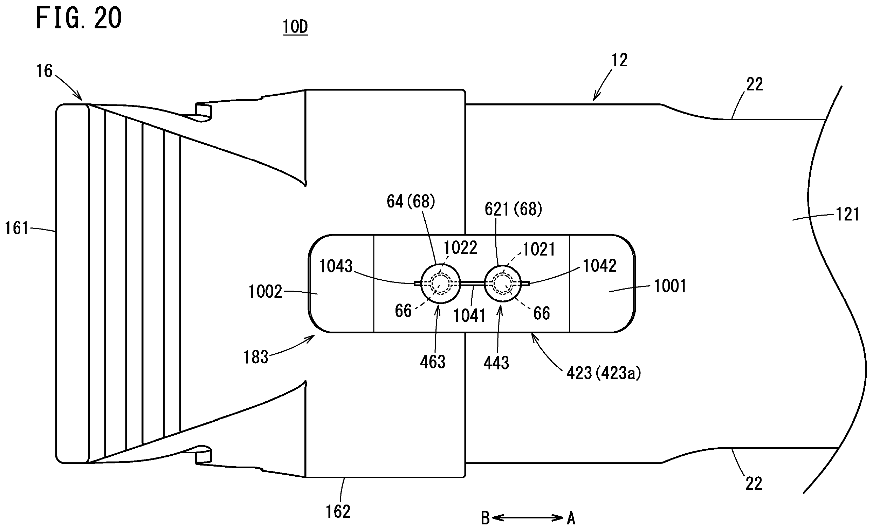

As shown in Fig. 19, a medicinal liquid administration device 10D according to a fourth embodiment includes a lock portion 183 that couples a cap 16 and a housing 12. As shown in Fig. 20, the lock portion 183 has a coupling member 423, a first coupling structure 443 that couples the housing 12 and the coupling member 423, and a second coupling structure 463 that couples the cap 16 and the coupling member 423.

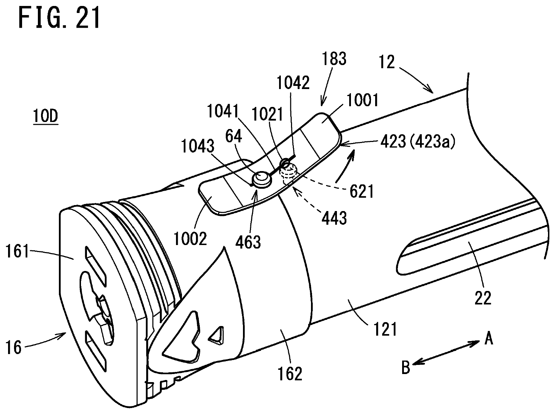

In a locked state in which the housing 12 and the cap 16 are coupled by the first and second coupling structures 443 and 463, the coupling member 423 is provided on a cap outer wall 162 and a housing outer wall 121, and is disposed across a proximal portion of the cap 16 and a distal portion of the housing 12. The coupling member 423 is provided detachably from at least one of the cap 16 and the housing 12. By detaching the coupling member 423 from either the cap 16 or the housing 12, an unlocked state is reached in which the cap 16 can be detached from the housing 12. The detaching direction of the coupling member 423 is a direction away from the housing outer wall 121 and the cap outer wall 162 (see Fig. 21). That is, the coupling member 423 is operated in a direction different from the axial direction of the housing 12 to bring the lock portion 183 into the unlocked state.

As shown in Fig. 20, the coupling member 423 is an elastically deformable plate-like member 423a. The plate-like member 423a is formed of, for example, rubber or a resin material. The coupling member 423 has a rectangular shape that is large in the longitudinal direction with respect to the width direction orthogonal to the longitudinal direction. The material of the plate-like member 423a is not limited to rubber and a resin material.

The coupling member 423 includes a first end portion 1001 provided at one end in the longitudinal direction and a second end portion 1002 provided at the other end in the longitudinal direction. The first end portion 1001 is disposed to face the housing outer wall 121 of the housing 12. The second end portion 1002 is disposed to face the cap outer wall 162 of the cap 16.

The coupling member 423 has a first engagement hole 1021 and a second engagement hole 1022. The first engagement hole 1021 and the second engagement hole 1022 are separated from each other in the longitudinal direction of the coupling member 423. The first engagement hole 1021 engages with a first engagement protrusion 621 of the housing outer wall 121. The first engagement hole 1021 has a smaller diameter than a head portion 68 of the first engagement protrusion 621. The first engagement hole 1021 is disposed closer to the first end portion 1001 with respect to the center of the coupling member 423 in the longitudinal direction. The second engagement hole 1022 engages with a second engagement protrusion 64 of the cap outer wall 162. The second engagement hole 1022 has a smaller diameter than a head portion 68 of the second engagement protrusion 64. The second engagement hole 1022 is disposed closer to the second end portion 1002 with respect to the center of the coupling member 423 in the longitudinal direction.

The coupling member 423 includes first to third slits 1041, 1042, and 1043. Each of the first to third slits 1041, 1042, and 1043 extends along the longitudinal direction of the coupling member 423 and penetrates in the thickness direction of the coupling member 423. The first slit 1041 extends between the first engagement hole 1021 and the second engagement hole 1022, and connects the first engagement hole 1021 and the second engagement hole 1022.

The second slit 1042 is provided in the opposite direction to the first slit 1041 with respect to the first engagement hole 1021. The second slit 1042 is formed to be connected to the first engagement hole 1021. The third slit 1043 is provided in the opposite direction to the first slit 1041 with respect to the second engagement hole 1022. The third slit 1043 is formed to be connected to the second engagement hole 1022. The coupling member 423 is not limited to including the second and third slits 1042 and 1043. For example, the coupling member 423 may include only the first slit 1041 between the first engagement hole 1021 and the second engagement hole 1022. In addition, the coupling member 423 may include only the second and third slits 1042 and 1043 without including the first slit 1041. Furthermore, the coupling member 423 may not include all of the first to third slits 1041, 1042, and 1043.

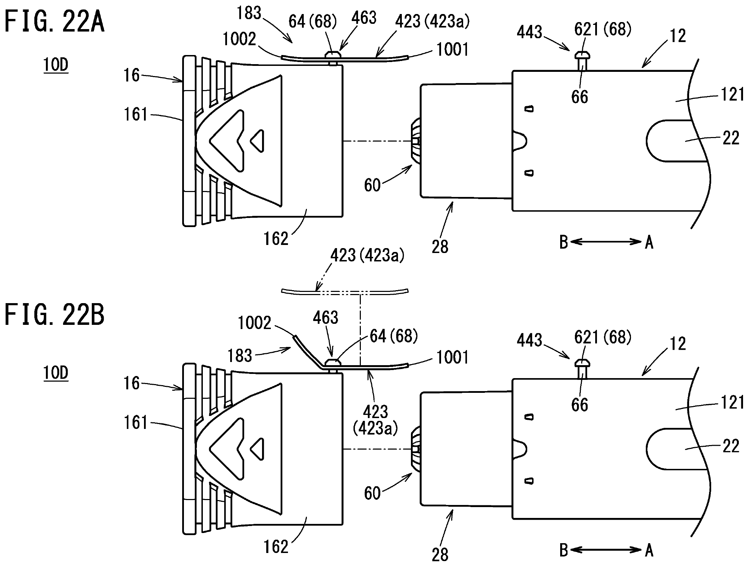

The first coupling structure 443 is an engagement structure in which the first engagement protrusion 621 and the first engagement hole 1021 engage with each other. As shown in Fig. 22A, by disengaging the coupling member 423 from the housing 12, the first engagement protrusion 621 disengages from the first engagement hole 1021, and a first coupling released state is reached in which the coupling state by the first coupling structure 443 is released. The second coupling structure 463 is an engagement structure in which the second engagement protrusion 64 and the second engagement hole 1022 engage with each other. As shown in Fig. 22B, by disengaging the coupling member 423 from the cap 16, the second engagement protrusion 64 disengages from the second engagement hole 1022, and a second coupling released state is reached in which the coupling state by the second coupling structure 463 is released.

When the coupling state between the housing 12 and the coupling member 423 by the first coupling structure 443 of the lock portion 183 is released, a user grips the first end portion 1001 and pulls up the coupling member 423 in a direction away from the housing outer wall 121 as shown in Fig. 21. By separating the first end portion 1001 of the coupling member 423 from the housing 12, the first engagement hole 1021 is expanded by the head portion 68 of the first engagement protrusion 621 through the first and second slits 1041 and 1042. When the head portion 68 of the first engagement protrusion 621 passes through the first engagement hole 1021 that has been expanded, the first engagement protrusion 621 disengages from the first engagement hole 1021. As shown in Fig. 22A, the first coupling released state is reached in which the coupling state between the housing 12 and the cap 16 by the first coupling structure 443 is released. By moving the cap 16 in the distal-end direction, the cap 16 is detached from the distal end of the housing 12. In this case, the coupling member 423 is detached from the housing 12 together with the cap 16 by the second coupling structure 463.

As shown in Fig. 22B, when the coupling states by the first coupling structure 443 and the second coupling structure 463 of the lock portion 183 are released, the user pulls up the first end portion 1001 of the coupling member 423 in a direction away from the housing outer wall 121 to release the engagement state of the first engagement protrusion 621 with the first engagement hole 1021, and then pulls up the second end portion 1002 of the coupling member 423 in a direction away from the cap 16. The second engagement hole 1022 is expanded by the head portion 68 of the second engagement protrusion 64 through the first and third slits 1041 and 1043. When the head portion 68 of the second engagement protrusion 64 passes through the second engagement hole 1022 that has been expanded, the second engagement protrusion 64 disengages from the second engagement hole 1022. Therefore, the second coupling released state is reached in which the coupling state between the coupling member 423 and the cap 16 by the second coupling structure 463 is released. The coupling member 423 is detached from the housing 12 and the cap 16.

When both the coupling states by the first coupling structure 443 and the second coupling structure 463 are released, the order of the release of the coupling of the first coupling structure 443 and the release of the coupling of the second coupling structure 463 is not particularly limited. For example, first, the coupling between the coupling member 423 and the cap 16 by the second coupling structure 463 may be released, and then the coupling between the coupling member 423 and the housing 12 by the first coupling structure 443 may be released.

The lock portion 183 is not limited to the case of releasing the coupling state by the first coupling structure 443. Only the coupling state between the coupling member 423 and the cap 16 by the second coupling structure 463 may be released while maintaining the coupling state by the first coupling structure 443. In this case, in an unlocked state in which the cap 16 can be detached from the housing 12, the coupling member 423 remains on the housing 12 in a state of being coupled to the housing 12.

The fourth embodiment has the following effects.

As shown in Fig. 20, the coupling member 423 is the plate-like member 423a that is provided across the proximal portion of the cap 16 and the distal portion of the housing 12 and is detachable from at least one of the cap 16 and the housing 12. By detaching the plate-like member 423a from either the cap 16 or the housing 12, an unlocked state is reached in which the cap 16 can be detached from the housing 12. Therefore, the coupling member 423 can be constituted with a simple configuration, and thus the cost can be reduced.

The coupling member 423 is the plate-like member 423a having the first engagement hole 1021 with which the first engagement protrusion 621 engages, and the second engagement hole 1022 with which the second engagement protrusion 64 engages. The first coupling structure 443 is an engagement structure in which the first engagement protrusion 621 and the first engagement hole 1021 engage with each other, and the second coupling structure 463 is an engagement structure in which the second engagement protrusion 64 and the second engagement hole 1022 engage with each other.

Therefore, the coupling between the coupling member 423 and the housing 12 by the first coupling structure 443 can be released by detaching the coupling member 423 from the housing 12, and the coupling between the coupling member 423 and the cap 16 by the second coupling structure 463 can be released by detaching the coupling member 423 from the cap 16. Accordingly, the user can select the handling of the coupling member 423 after the coupling state by the lock portion 183 is released.

As shown in Fig. 21, in an unlocked state in which the housing 12 and the cap 16 can be detached, the coupling state of one (first coupling structure 443) of the first coupling structure 443 and the second coupling structure 463 is released, and the coupling state of the other (second coupling structure 463) of the first coupling structure 443 and the second coupling structure 463 is maintained. Therefore, by maintaining the coupling state of the other of the first and second coupling structures 443 and 463, it is possible to select whether to detach and discard the coupling member 423 together with the cap 16 or to leave the coupling member 423 attached to the housing 12.

As shown in Fig. 22B, in the unlocked state, both the coupling states by the first coupling structure 443 and the second coupling structure 463 are released, and the first coupling released state and the second coupling released state are reached. Therefore, when the cap 16 is detached from the housing 12, the coupling member 423 can be prevented from remaining on either the cap 16 or the housing 12.

As shown in Fig. 20, the coupling member 423 has the first slit 1041 extending between the first engagement hole 1021 and the second engagement hole 1022, and the first slit 1041 connects the first engagement hole 1021 and the second engagement hole 1022. Therefore, when the first engagement protrusion 621 is disengaged from the first engagement hole 1021, the first slit 1041 facilitates the disengagement of the first engagement protrusion 621, making it easier to detach the coupling member 423. When the second engagement protrusion 64 is disengaged from the second engagement hole 1022, the first slit 1041 facilitates the disengagement of the second engagement protrusion 64, making it easier to detach the coupling member 423.

The coupling member 423 includes the second slit 1042 provided in the opposite direction to the first slit 1041 with respect to the first engagement hole 1021, and the third slit 1043 provided in the opposite direction to the first slit 1041 with respect to the second engagement hole 1022. The second slit 1042 is formed to be connected to the first engagement hole 1021, and the third slit 1043 is formed to be connected to the second engagement hole 1022.

Therefore, by providing the second and third slits 1042 and 1043 in addition to the first slit 1041, it is possible to more effectively disengage the first and second engagement protrusions 621 and 64 from the first and second engagement holes 1021 and 1022. Accordingly, the coupling member 423 is more easily detached.

As shown in Fig. 21, the coupling member 423 is operated in a direction different from the axial direction of the housing 12, and thus the lock portion 183 enters the unlocked state. Therefore, since the operation direction of the coupling member 423 is different from the axial direction of the housing 12, the coupling member 423 is less likely to be erroneously operated by the user.

In addition, the coupling member 423 is not limited to being formed of the plate-like member 423a that can engage with the first and second engagement protrusions 621 and 64. For example, the coupling member 423 may be a seal-like coupling member bonded across the distal portion of the housing 12 and the proximal portion of the cap 16. By peeling off the coupling member from at least one of the distal portion of the housing 12 and the proximal portion of the cap 16, an unlocked state can be reached in which the housing 12 and the cap 16 can be detached.

Note that the present invention is not limited to the above disclosure and can take various configurations without departing from the gist of the present invention.

## Claims

1. A medicinal liquid administration device for administering a medicinal liquid into a living body, the medicinal liquid administration device comprising:
a housing that is formed in a hollow cylindrical shape;
a syringe having a cylindrical body that is accommodated in the housing and filled with the medicinal liquid, and a puncture needle that communicates with the cylindrical body and administers the medicinal liquid into a living body; and
a cap that is provided at a distal end of the housing and detached when the puncture needle punctures a puncture target,
wherein the medicinal liquid administration device includes a lock portion coupling the cap and the housing,
the lock portion includes a coupling member,
a first coupling structure coupling the housing and the coupling member, and
a second coupling structure coupling the cap and the coupling member, and
by releasing a coupling state of at least one of the first coupling structure and the second coupling structure, an unlocked state is reached in which the cap can be detached from the housing.

2. The medicinal liquid administration device according to claim 1,
wherein at least a part of the coupling member is operated in a direction different from an axial direction of the housing to bring the lock portion into the unlocked state.

3. The medicinal liquid administration device according to claim 1 or 2,
wherein the unlocked state is reached by releasing a coupling state between the housing and the coupling member by the first coupling structure, and
when the cap is detached from the housing, the coupling member is detached from the housing together with the cap in a state in which the cap and the coupling member are coupled by the second coupling structure.

4. The medicinal liquid administration device according to claim 1,
wherein the coupling member is a rotary member formed in a cylindrical shape and provided rotatably about an axis of the housing, and the unlocked state is reached by rotating the rotary member.

5. The medicinal liquid administration device according to claim 2,
wherein the coupling member is formed in a hollow cylindrical shape having a distal end opening and a proximal end opening, the cap being inserted into the distal end opening and the housing being inserted into the proximal end opening, and includes an internal space in which the cap and the housing are accommodated,
the coupling member has a first rib protruding from an inner wall of a distal portion having the distal end opening, and
an engagement groove provided on an inner wall of a proximal portion having the proximal end opening and extending in a circumferential direction,
the cap has a second rib provided at a proximal portion and protruding from a cap outer wall,
a distal portion of the housing has an engagement protrusion protruding from a housing outer wall,
the first coupling structure is an engagement structure in which the engagement protrusion and the engagement groove engage with each other,
the second coupling structure is an abutment structure in which a proximal portion of the first rib and a distal portion of the second rib face each other in the axial direction of the housing, and the proximal portion and the distal portion can abut on each other, and
by rotating the coupling member relative to the housing about an axis of the housing, the engagement protrusion of the housing disengages from the engagement groove of the coupling member, and a first coupling released state is reached in which a coupling state of the first coupling structure is released.

6. The medicinal liquid administration device according to claim 5,
wherein the engagement groove of the coupling member has a spiral shape centered on an axis of the coupling member,
when the coupling state of the first coupling structure is released, the engagement protrusion moves along the engagement groove, so that the coupling member moves relative to the housing in a distal-end direction, and the proximal portion of the first rib and the distal portion of the second rib in the second coupling structure are separated from each other, and
when the cap is detached from the housing in the distal-end direction, the proximal portion of the first rib and the distal portion of the second rib abut on each other.

7. The medicinal liquid administration device according to claim 5,
wherein the housing has a first indicator,
the coupling member has a second indicator, and
by rotating the coupling member relative to the housing about the axis of the housing, the first indicator and the second indicator are linearly disposed in the axial direction of the housing and the coupling member,
the engagement protrusion of the housing disengages from the engagement groove of the coupling member, and the first coupling released state is reached in which the coupling by the first coupling structure is released.

8. The medicinal liquid administration device according to claim 7,
wherein the first indicator is a window portion through which the syringe can be visually confirmed from outside of the housing, and
the second indicator is a hole portion penetrating an outer wall of the coupling member.

9. The medicinal liquid administration device according to claim 5,
wherein the coupling member has a groove forming portion including the engagement groove, at least the groove forming portion is transparent, and the engagement groove can be visually confirmed from outside of the coupling member through the groove forming portion.

10. The medicinal liquid administration device according to claim 6,
wherein in the first coupling released state in which the engagement protrusion of the housing disengages from the engagement groove of the coupling member and the first coupling structure is released, the cap has an abutment portion that abuts on the distal portion of the coupling member.

11. The medicinal liquid administration device according to claim 1,
wherein the coupling member is a rotary member that is provided across a proximal portion of the cap and a distal portion of the housing and is rotatable about a vertical axis perpendicular to an axial direction of the housing, and the unlocked state is reached by rotating the rotary member.

12. The medicinal liquid administration device according to claim 2,
wherein in a state in which the housing and the cap are coupled by the first coupling structure and the second coupling structure, the coupling member is provided across a proximal portion of the cap and a distal portion of the housing,
the coupling member is provided on a cap outer wall to be rotatable about a vertical axis perpendicular to the axial direction of the housing,
the distal portion of the housing has a first engagement protrusion protruding from a housing outer wall,
the coupling member has a first engagement groove with which the first engagement protrusion engages, and the first engagement groove extends along a rotation direction about the vertical axis,
the first coupling structure is an engagement structure between the first engagement protrusion and the first engagement groove,
the second coupling structure is a fixing structure between the coupling member and the cap, and
by rotating the coupling member about the vertical axis, the first engagement protrusion disengages from the first engagement groove, and a first coupling released state is reached in which the first coupling structure is released.

13. The medicinal liquid administration device according to claim 12,
wherein the cap has a second engagement protrusion protruding from the cap outer wall,
the coupling member has a second engagement groove with which the second engagement protrusion engages, and the second engagement groove extends along the rotation direction about the vertical axis, and
by rotating the coupling member about the vertical axis, the second engagement protrusion moves along the second engagement groove.

14. The medicinal liquid administration device according to claim 13,
wherein by rotating the coupling member about the vertical axis, the second engagement protrusion disengages from the second engagement groove, and a second coupling released state is reached in which the coupling between the coupling member and the cap by the second coupling structure is released.

15. The medicinal liquid administration device according to claim 1,
wherein the coupling member is a slide member that is provided across a proximal portion of the cap and a distal portion of the housing and is slidable in an intersecting direction intersecting an axial direction of the housing, and the unlocked state is reached by moving the slide member in the intersecting direction.

16. The medicinal liquid administration device according to claim 2,
wherein in a state in which the housing and the cap are coupled by the first coupling structure and the second coupling structure, the coupling member is provided across a proximal portion of the cap and a distal portion of the housing,
the coupling member is provided on a cap outer wall to be slidable in an intersecting direction that intersects the axial direction of the housing and is along a housing outer wall,
the distal portion of the housing has a first engagement protrusion protruding from the housing outer wall,
the coupling member has a first engagement groove with which the first engagement protrusion engages, and the first engagement groove extends along the intersecting direction,
the first coupling structure is an engagement structure between the first engagement protrusion and the first engagement groove,
the second coupling structure is a fixing structure between the coupling member and the cap, and
by moving the coupling member in the intersecting direction, the first engagement protrusion disengages from the first engagement groove, and a first coupling released state is reached in which the first coupling structure is released.

17. The medicinal liquid administration device according to claim 16,
wherein the cap has a second engagement protrusion protruding from the cap outer wall,
the coupling member has a second engagement groove with which the second engagement protrusion engages, and the second engagement groove extends along the intersecting direction, and
by moving the coupling member in the intersecting direction, the second engagement protrusion moves along the second engagement groove.

18. The medicinal liquid administration device according to claim 17,
wherein by moving the coupling member in the intersecting direction, the second engagement protrusion disengages from the second engagement groove, and a second coupling released state is reached in which the coupling between the coupling member and the cap by the second coupling structure is released.

19. The medicinal liquid administration device according to claim 1,
wherein the coupling member is a plate-like member that is provided across a proximal portion of the cap and a distal portion of the housing and is detachable from at least one of the cap and the housing, and the unlocked state is reached by detaching the plate-like member from one of the cap and the housing.

20. The medicinal liquid administration device according to claim 2,
wherein a distal portion of the housing has a first engagement protrusion protruding from a housing outer wall,
a proximal portion of the cap has a second engagement protrusion protruding from a cap outer wall,
in a state in which the housing and the cap are coupled by the first coupling structure and the second coupling structure, the coupling member is provided across the proximal portion of the cap and the distal portion of the housing,
the coupling member is a plate-like member having a first engagement hole with which the first engagement protrusion engages and a second engagement hole with which the second engagement protrusion engages,
the first coupling structure is an engagement structure in which the first engagement protrusion and the first engagement hole engage with each other,
the second coupling structure is an engagement structure in which the second engagement protrusion and the second engagement hole engage with each other, and
by disengaging the coupling member from the housing, the first engagement protrusion disengages from the first engagement hole, and a first coupling released state is reached in which the coupling state by the first coupling structure is released, or by disengaging the coupling member from the cap, the second engagement protrusion disengages from the second engagement hole, and a second coupling released state is reached in which the coupling state by the second coupling structure is released.

21. The medicinal liquid administration device according to claim 20,
wherein in the unlocked state, the coupling state of one of the first coupling structure and the second coupling structure is released, and the coupling state of the other of the first coupling structure and the second coupling structure is maintained.

22. The medicinal liquid administration device according to claim 20,
wherein in the unlocked state, both the coupling states by the first coupling structure and the second coupling structure are released, and the first coupling released state and the second coupling released state are reached.

23. The medicinal liquid administration device according to claim 20,
wherein the coupling member has a first slit extending between the first engagement hole and the second engagement hole, and
the first slit connects the first engagement hole and the second engagement hole.

24. The medicinal liquid administration device according to claim 23,
wherein the coupling member includes a second slit provided in an opposite direction to the first slit with respect to the first engagement hole, and
a third slit provided in an opposite direction to the first slit with respect to the second engagement hole, and
the second slit is formed to be connected to the first engagement hole, and the third slit is formed to be connected to the second engagement hole.
